# EUROPEAN PATENT APPLICATION

(11) **EP 1 371 660 A1**
(43) Date of publication of application: **17.12.2003**
(21) Application number: 02380126.9
(22) Date of filing: 14.06.2002
(51) Int. Cl.: C07K 14/16, C07K 16/10, C12N 15/00

(54) **Vaccine**

(71) Applicant: CONSEJO SUPERIOR DE INVESTIGACIONES CIENTIFICAS, 28006 Madrid (ES); Pharmacia Spain, 08190 Sant Cugat Del Valles (ES)
(72) Inventor: Toran, Jose Luis, 28410 Manzanares del Real, Madrid (ES); Martinez Alonso, Carlos, 28034 Madrid (ES)
(74) Representative: Del Santo Abril, Natividad

(57) **Abstract**

The application relates to antibodies and fragments capable of binding HIV-1 gp120 protein, nucleic acids encoding such proteins, to the use of such proteins to identify active compounds, and to the use of the compounds as vaccines.

SEQUENCE LISTING

<110> Consejo Superior de Investigaciones Científicas
      Pharmacia Co.

<120> Vaccine

<130> p704912gb/de

<140>
<141>

<160> 43

<170> PatentIn Ver. 2.1

<210> 1
<211> 647
<212> DNA
<213> Homo sapiens

<220>
<221> Light chain

<400> 1
gagctcaccc agtctccgtc ctccctgtct gcatctgttg gagacagagt caccatcact   60
tgccgggcaa gtcagggcat tagagatgat ttaggctggt atcagcagaa accagggaaa  120
gcccctaagc gcctgatcta tgctgcatcc aatttacaaa gtggggtccc atcaaggttc  180
agcggcggcg gatctgggac agaattcact ctcacaatca gcagcctgca gcctgaagat  240
tttgcaactt attactgtct acagcataat agttaccccc tcactttcgg cggagggacc  300
aaggtggaga tcaaacgaac tgtggctgca ccatctgtct tcatcttccc gccatctgat  360
gagcagttga aatctggaac tgcctctgtt gtgtgcctgc tgaataactt ctatcccaga  420
gaggccaaag tacagtggaa ggtggataac gccctccaat cgggtaactc ccaggagagt  480
gtcacagagc aggacagcaa ggacagcacc tacagcctca gcagcaccct gacgctgagc  540
aaagcagact acgagaaaca caaagtctac gcctgcgaag tcacccatca gggcctgagt  600
tcgcccgtca caaagagctt caacaagggg aaagtgttaa ttctaga                647


<210> 2
<211> 215
<212> PRT
<213> Homo sapiens

<220>
<221> Light chain

<400> 2

Glu Leu Thr Gln Ser Pro Ser Ser Leu Ser Ala Ser Val Gly Asp Arg
  1               5                  10                  15

Val Thr Ile Thr Cys Arg Ala Ser Gln Gly Ile Arg Asp Asp Leu Gly
             20                  25                  30

Trp Tyr Gln Gln Lys Pro Gly Lys Ala Pro Lys Arg Leu Ile Tyr Ala
         35                  40                  45

Ala Ser Asn Leu Gln Ser Gly Val Pro Ser Arg Phe Ser Gly Gly Gly
     50                  55                  60

Ser Gly Thr Glu Phe Thr Leu Thr Ile Ser Ser Leu Gln Pro Glu Asp
65                  70                  75                  80

Phe Ala Thr Tyr Tyr Cys Leu Gln His Asn Ser Tyr Pro Leu Thr Phe
                85                  90                  95

Gly Gly Gly Thr Lys Val Glu Ile Lys Arg Thr Val Ala Ala Pro Ser
            100                 105                 110

Val Phe Ile Phe Pro Pro Ser Asp Glu Gln Leu Lys Ser Gly Thr Ala
        115                 120                 125

Ser Val Val Cys Leu Leu Asn Asn Phe Tyr Pro Arg Glu Ala Lys Val
    130                 135                 140

Gln Trp Lys Val Asp Asn Ala Leu Gln Ser Gly Asn Ser Gln Glu Ser
145                 150                 155                 160

Val Thr Glu Gln Asp Ser Lys Asp Ser Thr Tyr Ser Leu Ser Ser Thr
                165                 170                 175

Leu Thr Leu Ser Lys Ala Asp Tyr Glu Lys His Lys Val Tyr Ala Cys
            180                 185                 190

Glu Val Thr His Gln Gly Leu Ser Ser Pro Val Thr Lys Ser Phe Asn
        195                 200                 205

Lys Gly Lys Val Leu Ile Leu
    210                 215


<210> 3
<211> 675
<212> DNA
<213> Homo sapiens

<220>
<221> S8 Heavy chain

<400> 3
ctcgagtcgg ggggaggctt ggtaaagcct ggggggtccc ttagactctc ctgtgcagcc    60
tctggtttca ctttcagtag ctatgctatg cactgggtcc gccaggctcc aggcaagggg   120
ctggagtggg tggcatttat atggtttgat ggaagtaatg aacgatatgc agactccgtg   180
aagggccgat tcaccatcac cagagacaat cccaagaaca ctctctatct gcaaatgaac   240
agcctgagag tcgaggacac ggctgtttat tactgtgtga gaaggggagg ctcgattttg   300
actggttttc atttagacta ctggggccag ggaaccctgg tcaccgtctc ctcagcctcc   360
accaagggcc catcggtctt ccccctggca ccctcctcca agagcacctc tgggggcaca   420
gcggccctgg gctgcctggt caaggactac ttccccgaac cggtgacggt gtcgtggaac   480
tcaggcgccc tgaccagcgg cgtgcacacc ttcccggctg tcctacagtc ctcaggactc   540
tactccctca gcagcgtggt gaccgtgccc tccagcagct tgggcaccca gacctacatc   600
tgcaacgtga atcacaagcc cagcaacacc aaggtggaca agagagttga gcccaaatct   660
tgtgacaaaa ctagt                                                    675


<210> 4
<211> 225
<212> PRT
<213> Homo sapiens

<220>
<221> S8 Heavy chain

<400> 4
Leu Glu Ser Gly Gly Gly Leu Val Lys Pro Gly Gly Ser Leu Arg Leu
  1               5                  10                  15

Ser Cys Ala Ala Ser Gly Phe Thr Phe Ser Ser Tyr Ala Met His Trp
             20                  25                  30

Val Arg Gln Ala Pro Gly Lys Gly Leu Glu Trp Val Ala Phe Ile Trp
        35                  40                  45

Phe Asp Gly Ser Asn Glu Arg Tyr Ala Asp Ser Val Lys Gly Arg Phe
    50                  55                  60

Thr Ile Thr Arg Asp Asn Pro Lys Asn Thr Leu Tyr Leu Gln Met Asn
65                  70                  75                  80

Ser Leu Arg Val Glu Asp Thr Ala Val Tyr Tyr Cys Val Arg Arg Gly
                85                  90                  95

Gly Ser Ile Leu Thr Gly Phe His Leu Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Arg Val Glu Pro Lys Ser Cys Asp Lys Thr
    210                 215                 220

Ser
225

<210> 5
<211> 675
<212> DNA
<213> Homo sapiens

<220>
<221> S19 Heavy chain

<400> 5
ctcgagtcgg ggggaggcgt ggtccagccc gggaggtccc tgagactctc ctgtgcagca    60
tctggattca gcttcagtag tcatggcatg cactgggtcc gccaggctcc aggcaagggg   120
ctggagtggg tggcatttat atggtttgat ggaagtaatg aacgatatgc agactccgtg   180
aagggccgat tcaccatcac cagagacaat cccaagaaca ctctctatct gcaaatgaac   240
agcctgagag tcgaggacac ggctgtttat tactgtgtga gaaggggagg ctcgattttg   300
actggttttc atttagacta ctggggccag ggaaccctgg tcaccgtctc ctcagcctcc   360
accaagggcc catcggtctt ccccctggca ccctcctcca agagcacctc tgggggcaca   420
gcggccctgg gctgcctggt caaggactac ttccccgaac cggtgacggt gtcgtggaac   480
tcaggcgccc tgaccagcgg cgtgcacacc ttcccggctg tcctacagtc ctcaggactc   540
tactccctca gcagcgtggt gaccgtgccc tccagcagct tgggcaccca gacctacatc   600
tgcaacgtga atcacaagcc cagcaacacc aaggtggaca agagagttga gcccaaatct   660
tgtgacaaaa ctagt                                                    675

<210> 6

<211> 225
<212> PRT
<213> Homo sapiens

<220>
<221> S19 Heavy chain

<400> 6
Leu Glu Ser Gly Gly Gly Val Val Gln Pro Gly Arg Ser Leu Arg Leu
1               5                   10                  15

Ser Cys Ala Ala Ser Gly Phe Ser Phe Ser Ser His Gly Met His Trp
            20                  25                  30

Val Arg Gln Ala Pro Gly Lys Gly Leu Glu Trp Val Ala Phe Ile Trp
        35                  40                  45

Phe Asp Gly Ser Asn Glu Arg Tyr Ala Asp Ser Val Lys Gly Arg Phe
    50                  55                  60

Thr Ile Thr Arg Asp Asn Pro Lys Asn Thr Leu Tyr Leu Gln Met Asn
65                  70                  75                  80

Ser Leu Arg Val Glu Asp Thr Ala Val Tyr Tyr Cys Val Arg Arg Gly
                85                  90                  95

Gly Ser Ile Leu Thr Gly Phe His Leu Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Arg Val Glu Pro Lys Ser Cys Asp Lys Thr
    210                 215                 220

Ser
225


<210> 7
<211> 675
<212> DNA
<213> Homo sapiens

<220>
<221> S20 Heavy chain

<400> 7
ctcgagtcgg ggggaggcgt ggtccagcct gggaggtccc tgagactttc ctgctcagcc    60

tctggattca gcttcagaga ttatgccatg cactgggtcc gccaggctcc aggcaagggg 120
ctggagtggg tggcatttat atggtttgat ggaagtaatg aacgatatgc agactccgtg 180
aagggccgat tcaccatcac cagagacaat cccaagaaca ctctctatct gcaaatgaac 240
agcctgagag tcgaggacac ggctgtttat tactgtgtga gaaggggagg ctcgattttg 300
actggttttc atttagacta ctggggccag ggaaccctgg tcaccgtctc ctcagcctcc 360
accaagggcc catcggtctt ccccctggca ccctcctcca agagcacctc tgggggcaca 420
gcggccctgg gctgcctggt caaggactac ttccccgaac cggtgacggt gtcgtggaac 480
tcaggcgccc tgaccagcgg cgtgcacacc ttcccggctg tcctacagtc ctcaggactc 540
tactccctca gcagcgtggt gaccgtgccc tccagcagct tgggcaccca gacctacatc 600
tgcaacgtga atcacaagcc cagcaacacc aaggtggaca agagagttga gcccaaatct 660
tgtgacaaaa ctagt 675

<210> 8
<211> 225
<212> PRT
<213> Homo sapiens

<220>
<221> S20 Heavy chain

<400> 8
Leu Glu Ser Gly Gly Gly Val Val Gln Pro Gly Arg Ser Leu Arg Leu
1               5                   10                  15

Ser Cys Ser Ala Ser Gly Phe Ser Phe Arg Asp Tyr Ala Met His Trp
            20                  25                  30

Val Arg Gln Ala Pro Gly Lys Gly Leu Glu Trp Val Ala Phe Ile Trp
        35                  40                  45

Phe Asp Gly Ser Asn Glu Arg Tyr Ala Asp Ser Val Lys Gly Arg Phe
    50                  55                  60

Thr Ile Thr Arg Asp Asn Pro Lys Asn Thr Leu Tyr Leu Gln Met Asn
65                  70                  75                  80

Ser Leu Arg Val Glu Asp Thr Ala Val Tyr Tyr Cys Val Arg Arg Gly
                85                  90                  95

Gly Ser Ile Leu Thr Gly Phe His Leu Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Arg Val Glu Pro Lys Ser Cys Asp Lys Thr
    210                 215                 220

Ser
225


<210> 9
<211> 12
<212> PRT
<213> Artificial sequence

<220>
<221> Description of Artificial Sequence: peptides capable of inhibiting
      binding of antibodies to gp120

<400> 9
Gly Ile Gln Leu Ala Asn Pro Pro Arg Leu Tyr Gly
  1               5                   10


<210> 10
<211> 12
<212> PRT
<213> Artificial sequence

<220>
<221> Description of Artificial Sequence: peptides capable of inhibiting
      binding of antibodies to gp120

<400> 10
Phe Leu Gln Pro Pro Asp Phe Ser His Leu Pro Pro
  1               5                   10


<210> 11
<211> 7
<212> PRT
<213> Artificial sequence

<220>
<221> Description of Artificial Sequence: peptides capable of inhibiting
      binding of antibodies to gp120

<400> 11
Ser Ala Met Glu Ala Pro Pro
  1               5


<210> 12
<211> 12
<212> PRT
<213> Artificial sequence

<220>
<221> Description of Artificial Sequence: peptides capable of inhibiting
      binding of antibodies to gp120

<400> 12
Leu Leu Ala Asp Thr Thr His His Arg Pro Trp Thr
  1               5                   10

<210> 13
<211> 12
<212> PRT
<213> Artificial sequence

<220>

<221> Description of Artificial Sequence: peptides capable of inhibiting
      binding of antibodies to gp120

<400> 13
Gln Glu Val Gly Lys Ala Met Tyr Ala Pro Pro Ile
  1               5                   10


<210> 14
<211> 12
<212> PRT
<213> Artificial sequence

<220>
<221> Description of Artificial Sequence: peptides capable to bind to
      antibodies

<400> 14
Ser Gly Leu Asp Gly Met His Val Asn Ser Pro Trp
  1               5                   10


<210> 15
<211> 12
<212> PRT
<213> Artificial sequence

<220>
<221> Description of Artificial Sequence: peptides capable to bind to
      antibodies

<400> 15
His Thr Lys Cys Ser Asp Ala Ser Cys Pro Leu Ile
  1               5                   10


<210> 16
<211> 12
<212> PRT
<213> Artificial sequence

<220>
<221> Description of Artificial Sequence: peptides capable to bind to
      antibodies

<400> 16
Ser Ala Lys Pro Ser Tyr Gln Pro Tyr Ala Gln Pro
  1               5                   10


<210> 17
<211> 12
<212> PRT
<213> Artificial sequence

<220>
<221> Description of Artificial Sequence: peptides capable to bind to
      antibodies

<400> 17
Phe Pro Ala Ser Met Pro Gly Leu Leu Leu Arg Val
  1               5                   10


<210> 18

<211> 12
<212> PRT
<213> Artificial sequence

<220>
<221> Description of Artificial Sequence: peptides capable to bind to
      antibodies

<400> 18
Gln Val Met Arg Met Met Pro Asn Gly Val Tyr Cys
  1               5                   10


<210> 19
<211> 12
<212> PRT
<213> Artificial sequence

<220>
<221> Description of Artificial Sequence: peptides capable to bind to
      antibodies

<400> 19
Gln Asp Arg Ala Leu Ile Thr Pro Leu Asp Gln Thr
  1               5                   10


<210> 20
<211> 12
<212> PRT
<213> Artificial sequence

<220>
<221> Description of Artificial Sequence: peptides capable to bind to
      antibodies

<400> 20
His Asp Glu Phe Val Trp Ile Ser Ile Trp Glu Pro
  1               5                   10


<210> 21
<211> 12
<212> PRT
<213> Artificial sequence

<220>
<221> Description of Artificial Sequence: peptides capable to bind to
      antibodies

<400> 21
Trp Thr Thr Asn Phe Ala Asp Pro Pro Ser Ser Thr
  1               5                   10


<210> 22
<211> 12
<212> PRT
<213> Artificial sequence

<220>
<221> Description of Artificial Sequence: peptides capable to bind to
      antibodies

<400> 22

Ser Ser Cys Ala Ala Phe Trp Ser Lys Ala Arg Pro
1               5                   10

<210> 23
<211> 12
<212> PRT
<213> Artificial sequence

<220>
<221> Description of Artificial Sequence: peptides capable to bind to
      antibodies

<400> 23
Cys Leu Ser Ser Asn Ser Ser Pro Pro Pro Arg Pro
1               5                   10

<210> 24
<211> 12
<212> PRT
<213> Artificial sequence

<220>
<221> Description of Artificial Sequence: peptides capable to bind to
      antibodies

<400> 24
His Thr Arg Val Leu Pro Ser Thr Ala Met Thr Leu
1               5                   10

<210> 25
<211> 12
<212> PRT
<213> Artificial sequence

<220>
<221> Description of Artificial Sequence: peptides capable to bind to
      antibodies

<400> 25
Leu Phe Gln Lys Gln Ile Glu Ser Pro Trp Arg Ser
1               5                   10

<210> 26
<211> 12
<212> PRT
<213> Artificial sequence

<220>
<221> Description of Artificial Sequence: peptides capable to bind to
      antibodies

<400> 26
His Gly His Pro Leu Lys Thr Asn Thr His Arg Ser
1               5                   10

<210> 27
<211> 12
<212> PRT

<213> Artificial sequence

<220>
<221> Description of Artificial Sequence: peptides capable to bind to
      antibodies

<400> 27
Met Pro Asn Pro Arg Gln Asn Pro Pro Pro Pro Leu
 1               5                   10


<210> 28
<211> 12
<212> PRT
<213> Artificial sequence

<220>
<221> Description of Artificial Sequence: peptides capable to bind to
      antibodies

<400> 28
Asn Phe Gln Thr Pro Asp Arg Thr Gln Ser Asn Leu
 1               5                   10


<210> 29
<211> 12
<212> PRT
<213> Artificial sequence

<220>
<221> Description of Artificial Sequence: peptides capable to bind to
      antibodies

<400> 29
Phe Tyr Thr Pro Thr Met His Ser Tyr Gly Ile Gln
 1               5                   10


<210> 30
<211> 12
<212> PRT
<213> Artificial sequence

<220>
<221> Description of Artificial Sequence: peptides capable to bind to
      antibodies

<400> 30
Ser Val Ser Pro Asn Met Arg Met Leu His Trp Trp
 1               5                   10


<210> 31
<211> 12
<212> PRT
<213> Artificial sequence

<220>
<221> Description of Artificial Sequence: peptides capable to bind to
      antibodies

<400> 31
Thr Thr Gly Asp His Arg Ala Phe Trp Leu Gly Gly
 1               5                   10

<210> 32
<211> 12
<212> PRT
<213> Artificial sequence

<220>
<221> Description of Artificial Sequence: peptides capable to bind to
      antibodies

<400> 32
Asn Tyr Phe Gln Gln Pro Pro Glu Arg His Ser Ser
  1               5                   10


<210> 33
<211> 12
<212> PRT
<213> Artificial sequence

<220>
<221> Description of Artificial Sequence: peptides capable to bind to
      antibodies

<400> 33
His Ile Glu Thr Leu Leu Pro Ala Pro Glu Leu Ser
  1               5                   10


<210> 34
<211> 12
<212> PRT
<213> Artificial sequence

<220>
<221> Description of Artificial Sequence: peptides capable to bind to
      antibodies

<400> 34
Lys Ala Pro Ile Pro Ser Ser Ile Pro Gly Phe Arg
  1               5                   10


<210> 35
<211> 12
<212> PRT
<213> Artificial sequence

<220>
<221> Description of Artificial Sequence: peptides capable to bind to
      antibodies

<400> 35
Gly Thr Thr Gln Asn Ala Met Ser Leu Ala Arg Leu
  1               5                   10


<210> 36
<211> 12
<212> PRT
<213> Artificial sequence

<220>

<221> Description of Artificial Sequence: peptides capable to bind to
      antibodies

<400> 36
Gln Pro Thr Thr Pro Phe Phe Asp Trp Asp Thr His


<210> 37
<211> 12
<212> PRT
<213> Artificial sequence

<220>
<221> Description of Artificial Sequence: peptides capable to bind to
      antibodies

<400> 37
His Ala Ser Thr Pro Ser Ser Pro Trp Ser Arg Pro
  1               5                   10


<210> 38
<211> 12
<212> PRT
<213> Artificial sequence

<220>
<221> Description of Artificial Sequence: peptides capable to bind to
      antibodies

<400> 38
Met Gln Ser Gln Leu Tyr Arg Asp Ser Pro Arg Gly
  1               5                   10


<210> 39
<211> 12
<212> PRT
<213> Artificial sequence

<220>
<221> Description of Artificial Sequence: peptides capable to bind to
      antibodies

<400> 39
Leu Pro Asn Ala Thr Lys Leu Ala Pro Ile Ser Pro
  1               5                   10


<210> 40
<211> 12
<212> PRT
<213> Artificial sequence

<220>
<221> Description of Artificial Sequence: peptides capable to bind to
      antibodies

<400> 40
Thr Ala Met Asn Leu Gly Pro Ala Leu Phe Arg Thr
  1               5                   10


<210> 41
<211> 12

<212> PRT
<213> Artificial sequence

<220>
<221> Description of Artificial Sequence: peptides capable to bind to
      antibodies

<400> 41
Trp Phe Lys Pro Pro Gln Thr Pro Leu Thr Leu Met
  1               5                   10


<210> 42
<211> 12
<212> PRT
<213> Artificial sequence

<220>
<221> Description of Artificial Sequence: peptides capable to bind to
      antibodies

<400> 42
Thr Met Gln Pro Tyr Lys Ser Trp Trp Ser Ser Lys
  1               5                   10


<210> 43
<211> 12
<212> PRT
<213> Artificial sequence

<220>
<221> Description of Artificial Sequence: peptides capable to bind to
      antibodies

<400> 43
Ala Asp Val Met Leu His Ser Lys His Val Gln Met
  1               5                   10

## Description

The application relates to antibodies and fragments, capable of binding HIV-1 gp120 protein, to nucleic acids encoding such proteins, to the use of such proteins to identify active compounds, and to the use of the compounds as vaccines.

HIV-1 induces CD4⁺ T lymphocyte depletion and a subsequent acquired immune deficiency syndrome (AIDS) in the host. Virus entry into host cells is mediated by viral envelope glycoproteins, the exterior 120 (gp120) and the transmembrane envelope glycoprotein 41 (gp41); gp120 represents the most exposed protein and forms a trimeric envelope protein spike on the virion. HIV-1 enters by direct fusion between the virion surface Env protein and the target cell, in a process that requires viral Env protein and two distinct cell surface receptor molecules, CD4 and a specific chemokine receptor (Berger, *et al.* (1999), Chemokine receptors as HIV-1 co-receptors: roles in viral entry, tropism, and disease. *Annu. Rev. Immunol.* **17**, 657-700). Although HIV-1 strain specificity to chemokine co-receptors is complex, CCR5 is used preferentially by most primary isolates and not by T c ell-adapted laboratory strains (TCLA); CXCR4 is used preferentially by laboratory strains and some primary isolates. CCR3 and CCR2b have also been reported as HIV-1 co-receptors.

Several chemokine ligands are reported to inhibit HIV-1 infection. CXCR4, used as co-receptor by T-tropic HIV-1 strains, can be blocked by SDF-1. HIV-1-infected patients with SDF-1β gene (SDF1 3'-A allele) variants are associated with slower progression to AIDS. Other CCR5 chemokine ligands such as RANTES, MIP-1α and MIP-1β were shown to inhibit M-tropic HIV- 1 infection of CD4⁺ cells. The role of CCR5 as an *in vivo* co-receptor is supported by resistance to R5 HIV-1 virus infection in individuals homozygous for a 32 bp CCR5 gene deletion (32-CCR5). Allele polymorphisms in the CCR5 promoter and CCR2 (CCR2-64I) genes are also reported to influence in the progression to AIDS.

Several lines of evidence suggest that gp120 interaction with target cell receptors involves initial binding to CD4; this induces conformational changes in gp120, enhancing co-receptor binding. The gp120 regions for co-receptor binding have been studied by antibody inhibition, mutagenesis and X-ray crystallography. Highly conserved Env residues important for co-receptor binding were localised on two beta strands in the gp120 bridging sheet minidomain. The nature of the gp120-receptor interaction associated with conformational changes in Env have important implications for antibody blocking of Env functions.

The inventors have performed exhaustive analyses of an HIV-1-infected LTNP (>15 years) individual, including HIV-1 chemokine genes associated with AIDS delay, characterisation of the LTNP HIV-1 virus and molecular analysis of the primary and secondary antibody response to the HIV-1 gp120 protein. Genotyping for CCR5, CCR5 promoter, CCR2b and 3UTR-SDF-1β showed no allele mutations known to be associated with AIDS delay. The LTNP isolate was classified as NSI virus by infection of MT-2 cells. LTNP virus *gp120* and *Nef* genes were analysed. Comparative analysis of multiple viral clones of the gp120 C2-V3-C3 region obtained from the donor, several Spanish HIV-1 isolates, and reference virus strains indicate that the donor isolate belongs to the B clade. According to virus phenotype, gp120 V3 regions display NSI/M-tropic markers. No premature stop codons or deletions were found in the *Nef* gene sequences from the LTNP virus. The humoral response to gp120 shows an IgM response comprised of low affinity polyreactive antibodies that mature to a more competent secondary IgG response. High affinity specific IgG Fabs to gp120 obtained from phage display libraries constructed from the donor were able to neutralise several reference viral strains *in vitro,* including X4 and R5 HIV-1 virus. One of the IgG Fabs tested (S8) showed *in vivo* neutralising activity against M-tropic (Bal) HIV-1 virus, using human PBL-reconstituted SCID mice as a viral infection model. Peptide mimotopes able to compete for Fab-gp120 binding were selected from random peptide phage display libraries. Mimopeptide information and molecular modeling of the gp120 structure were used to identify the S8 Fab epitope. The model suggests that the Fab epitope is conformational and involves key gp120 residues implicated in the chemokine co-receptor binding site. This epitope was found conserved in most HIV-1 virus. Fab S8 activity may involve interaction of a charged HCDR3 residue (Arg95) in this Fab with Glu381 in gp120, producing significant conformational changes in the gp120 inner-outer interdomain.

The information provided by the inventors has resulted in assays and compounds which are useful for studying and/or treating HIV-1 infections.

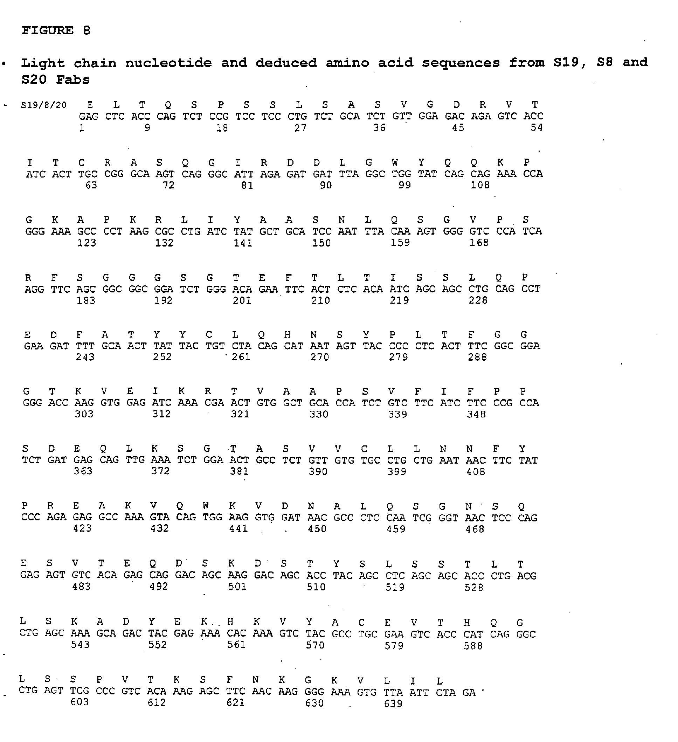

The first aspect of the invention provides an antibody or a fragment thereof, comprising a light chain and/or a heavy chain, the light chain or heavy chain comprising the amino acid sequence shown in SEQ ID 2, SEQ ID 4, SEQ ID 6 or SEQ ID 8 and/or shown in Figures 8 to 11.

Preferably the fragment of the antibody is capable of binding gp120 protein of human immunodeficiency virus (HIV), with the proviso that when part of SEQ ID 4, SEQ ID 6 or SEQ ID 8 are present, at least one amino acid from amino acid number 119 onwards of each sequence is present in the antibody fragment.

The amino acid sequence shown in SEQ ID 2 encodes the light chain for each of the 3 antibodies identified by the inventors, S8, S19 and S20. SEQ ID 4 encodes the heavy chain of S8, SEQ ID 6 encodes the heavy chain of S19 and SEQ ID 8 encodes the heavy chain of S20. These sequences are also shown in Figures 8 to 11.

Preferably the antibody or fragment, according to the invention, comprises an HCDR3 loop, the loop comprising Arg₉₅. This residue has been identified as being especially important in the interaction of the antibodies with gp120. It is believed to form an electrostatic interaction with Glu₃₈₁ of gp120. Most preferably the HCDR3 loop is that from Fab S8.

The antibody or fragment may therefore comprise an HCDR3 loop, the loop comprising an Arg residue that interacts electrostatically with gp120 on binding to it.

Preferably the antibodies or fragments thereof according to the first aspect of the invention comprise at least a fragment of a heavy chain encoded by SEQ ID 6 (S19 heavy chain) and include residue 32 of that peptide at least. This has been identified by the inventors as being involved in gp120 binding.

The antibody or fragment thereof may alternatively comprise the heavy chain of S20 (SEQ ID 8) and comprise Arg₃₀ and Asp₃₁, which are residues in the HCDR1 region and have been shown by the inventors to be involved in antigen binding. Preferably residues 30 or 31 were replaced by different amino acids to improve gp120 binding. This may be carried out by somatic mutation. Most preferably the changes are from Ser₃₀ and Ser₃₁ to Asp₃₀ and Arg₃₁.

The preferred substitutions for the S19 and S20 fragments are shown in detail in the article by Torán J.L. *et al.,* European Journal of Immunology, Vol. 31 (2001), pages 128-131.

The second aspect of the invention provides an antibody or a fragment thereof comprising a light chain and a heavy chain, the light chain comprising the amino acid sequence shown in SEQ ID 2 and the heavy chain comprising an amino acid sequence selected from SEQ ID 4, SEQ ID 6 and SEQ ID 8, the antibody or fragment being capable of binding gp120 protein from HIV.

Preferably the antibody fragments, according to the first or second aspects of the invention, are F(ab')₂, Fab or single chain (SchFv) fragments.

More preferably the antibody fragment used is the Fab S8 antibody.

Antibodies *per se* are well known in the art. They usually comprise so-called heavy chains and light chains. One light chain is usually attached to a heavy chain by means of a disulphide bond. Two heavy chains are in turn usually attached to each other by means of one or more disulphide bonds. T he antibodies may be one of several different c lasses o f antibody, such as IgG, IgD, IgE, IgA and IgM.

Preferably the antibodies are human antibodies or fragments. Alternatively, the antibodies may be derived from non-human sources and may preferably be humanised using techniques known in the art.

Most preferably, the antibody or fragment thereof, is a human IgG antibody or fragment thereof.

F(ab')₂ antibodies are formed by digesting antibodies comprising the two light chains and two heavy chains with pepsin. Fab fragments are formed by digesting antibodies comprising the two light chains and two heavy chains with papain to form two Fab fragments consisting of a fragment of heavy chain attached by at least one covalent bond, such as a disulphide bond to a light chain. The techniques for forming F(ab')₂ and Fab fragments from antibodies are well known in the art.

Preferably the HIV virus from which the gp120 protein is derived is HIV-1.

The sequences of gp120 are known to be slightly variable. For example, different sequences are shown in the articles by Myers, *et al.,* (1992); Gurgo, *et al.* (1998); and McCutchan, *et al.,* (1992).

Preferably, the sequence of the gp120 used in the invention is Human Immunodeficiency virus type 1 (HBx2) complete genome; HIV-1/LAV(IIIB) (Ratner, L. *et al.,* (1985)).

Preferably the antibodies or fragments thereof are capable of binding gp120 with a Kd of at least 1 x 10⁻¹⁰ M, especially greater than 9.0 x 10⁻¹⁰ M. Preferably the binding specificity is measured by surface plasmon resonance.

The third aspect of the invention provides a nucleic acid molecule selected from:
(a) a nucleic acid molecule which encodes for an antibody or a fragment of an antibody according to the invention;
(b) a nucleic acid molecule comprising the nucleic acid shown in SEQ ID 1, SEQ ID 3, SEQ ID 5 or SEQ ID 7, preferably the nucleic acid sequence comprises the sequence shown in SEQ ID 1 and additionally one or more of SEQ ID 3, SEQ ID 5 or SEQ ID 7;
(c) a nucleic acid molecule, the complementary strand of which hybridises to a nucleic acid molecule as defined in (a) or (b) and which encodes an antibody or a fragment of an antibody which is capable of binding gp120 protein from HIV, especially HIV-1, and which preferably encodes an antibody or a fragment of an antibody, having both heavy chain and light chains; and
(d) nucleic acid molecules which differ from the sequence of (c) due to the degeneracy of the genetic code.

Amino acids are encoded by triplets of three nucleotides of a certain sequence, so called codons. For most amino acids there is more than one codon. This is called "degeneracy". Hence, one or more triplets may be replaced by other triplets, but the nucleic acid molecule may still encode an identical peptide.

Nucleic acid molecules comprising a nucleotide sequence having greater than 90% homology, preferably 92, 94, 95, 96, 98 or 99% homology to SEQ ID 1, SEQ ID 3, SEQ ID 5 or SEQ ID 7 are also provided by the invention. Fragments of antibodies encoded by such nucleic acid molecules are also provided. Preferably the antibodies and fragments are capable of binding gp120 protein from HIV, most preferably HIV-1.

The nucleic acid s equences for the light chain, S8 heavy chain, S19 heavy chain and S20 heavy chain are also shown in Figures 8 to 11.

The nucleic acid sequences may be used in vaccines.

Vectors and host cells comprising nucleic acid molecules according to the invention are also provided. Suitable vectors include plasmids, cosmids and viral vectors. The vectors preferably comprise one or more regulatory sequences, such as promoters, termination and secretory signal sequences to enable to nucleic acid molecule, according to the invention, to be expressed as a protein. Preferably the vector is a retroviral vector, which may be used to infect cells or patients with the nucleic acid. Such retroviral vectors may be used for gene therapy purposes. Adenoviral vectors are especially preferred.

Suitable host cells include those known in the art including eukaryotic cells, such as mammalian cells, yeast cells and prokaryotic cells such as *E-coli,* in the art.

Preferably the nucleic acid molecule is DNA or RNA and preferably comprises naturally occurring nucleotides, for example containing adenine, guanine, thymine, cytosine or uracil as bases. Non-naturally occurring nucleotides, for example of the sort known in the art, may also be used.

The antibodies or fragments of antibodies, according to the invention, may be used to identify compounds capable of competing for the binding of the antibody or fragment thereof to HIV gp120 protein or a fragment thereof. Preferably the gp120 protein is from HIV-1. The fragment of a gp 120 protein may comprise a portion of the protein which contains one or both of Ile₄₂₀ - Gln₄₂₂ and/or Pro₄₃₇ - Pro₄₃₈. The chemical compound is preferably a peptide or a peptoid. In particular, the chemical compound may be a mimotope.

The mimotopes are preferably peptides that mimican epitope. The mimotopes may have amino acid sequences that bear no similarity with the amino acid sequence of the original epitope. In particular, the mimotopes may be identified by screening random peptide arrays.

By peptide, we mean a sequence of amino acids, which may be naturally or non-naturally occurring amino acids, of less than 40 or 35 amino acids, preferably less than 30, less than 25, less than 20, less than 15, especially less than 13 amino acids in length.

Amino acids are the basic building blocks from which peptides and proteins are constructed. Amino acids possess both an amino group (-NH₂) and a carboxyl group (-COOH). Many amino acids, but not all, have the structure NH₂-CHR-COOH, where R is hydrogen or any of a variety of functional groups. 20 amino acids are naturally genetically coded, however, non-naturally occurring amino acids, such as those known in the art, may be used.

A peptide is composed of a plurality of amino acid residues joined together by peptidyl (-NHCO-) bonds.

These may be produced by expression of the nucleic acid molecules of the invention or artificially by chemical synthesis.

Peptoids are analogues of a peptide in which one or more of the peptide bonds are replaced by pseudopeptide bonds, e.g.:
Carba ψ (CH₂-CH₂)
Depsi ψ (CO-O)
Hydroxyethylene ψ (CHOH-CH₂)
Ketomethylene ψ (CH-CH₂)
Methylene-ocy CH₂-O-
Reduced CH₂-NH
Thiomethylene CH₂-S-
Thiopeptide CS-NH
N-modified -NRCO-

By epitope we mean an immunologically active region or an immunogen that is capable of binding to the antibody or fragment thereof. Preferably the immunogen is gp120 from HIV, especially HIV-1, or a fragment of such a protein.

Preferably, random phage display libraries, or other such combinatorial libraries, may be used to identify chemical compounds that can complete for the binding of the antibody or fragment thereof to the HIV gp120 protein or a fragment of the protein.

The inventors have found a number of peptide sequences which are capable of competing for the binding of the antibody or fragment thereof to HIV gp120 protein.

The invention also includes chemical compounds identifiable by the methods described above. Preferably the chemical compound is a peptide or peptoid. The peptide may especially be a conformational epitope to one or both of regions Ile₄₂₀ - Gln₄₂₂ and/or Pro₄₃₇ - Pro₄₃₈ of the gp120 protein. The peptide may comprise an amino acid sequence selected from SEQ ID 9, SEQ ID10, SEQ ID 11, SEQ ID 12 and SEQ ID 13, or a sequence shown in any one of SEQ ID Nos. 14 to 43, or a sequence shown in Table 1.

More preferably the peptides may comprise an amino acid sequence as shown in both SEQ ID 9 and SEQ ID 10.

Nucleic acid molecules encoding the peptides are also provided by the invention.

The chemical compounds, such as the peptides or peptoids, may be used to produce a vaccine. Nucleic acids, such as DNA, encoding the peptides may also be used as vaccines. These latter vaccines are usually known by the general term "DNA vaccines". Alternatively the nucleic acid may be within a vector, such as a retroviral vector.

Preferably the compounds are mixed with one or more adjuvants such as bovine serum albumin, aluminium potassium sulphate, Freund's incomplete adjuvant or Freund's complete adjuvant.

The vaccine may be administered in a dose of typically 0.01 - 50 mg/kg., especially 0.1 - 5 mg/kg. It may be administered by techniques known in the art, including intravenously, intradermally, subcutaneously, intramuscularly, or intraperitoneally.

The invention also includes within its scope the use of antibodies or fragments according to the invention or compounds according to the invention, for the prevention or treatment of HIV, especially HIV-1, infections. The invention also includes antibodies or fragments thereof, according to the invention, or compounds according to the invention, for use to treat HIV, especially HIV-1, infections.

The antibodies or fragments according to the invention or compounds according to the invention may be labelled, e.g. with fluorescent compounds, radioactive nucleotides, colloidal metals, bioluminescent compounds and/or enzymes. Such labels are well known in the art. The antibodies or fragments or compounds may then be used to study HIV infections *in vivo* or *in vitro* by their ability to bind to gp120 or to inhibit the binding of antibodies, or fragments, to the gp120 protein.

The antibodies, fragments or chemical compounds may also be used to inhibit the binding of HIV to viral co-receptors. The inventors have noted that the antibodies according to the invention interact with Ile₄₂₀ - Gln₄₂₂ and/or Pro₄₃₇ - Pro₄₃₈ of the gp120 protein of HIV. This has been noted by C. Rizzuto and J. Sodroski (2000) as being within a region that is important for binding.

In a further aspect of the invention the antibodies, fragments or compounds may be used to evaluate AIDS progression and/or the state of infection as a prognosis marker.

A still further aspect of the invention provides a kit for studying HIV infection, especially HIV-1 infection, *in vivo* and/or *in vitro,* comprising an antibody or a fragment, or a compound according to the invention.

The antibodies, fragments or compounds may be labelled as already indicated.

A still further aspect of the invention provides a kit for studying the inhibition of the binding of HIV to a co-receptor comprising the use of an antibody, fragment or a compound according to the invention. Preferably the HIV is HIV-1. Preferably the interaction that is inhibited is the interaction between gp120 and CCR5.

The invention will now be described by means of example only with reference to the following figures:

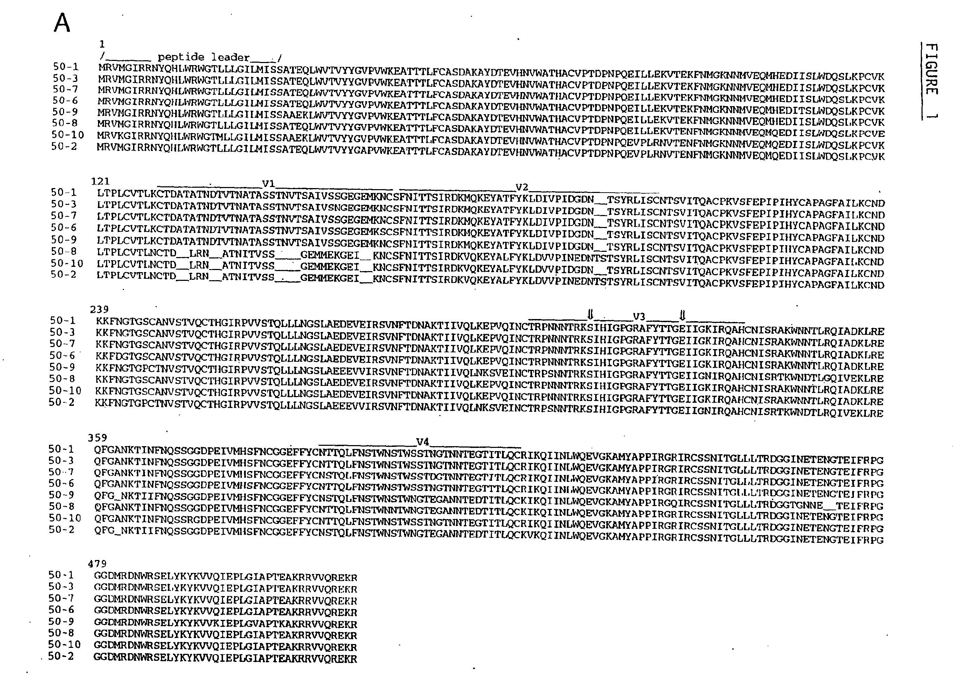

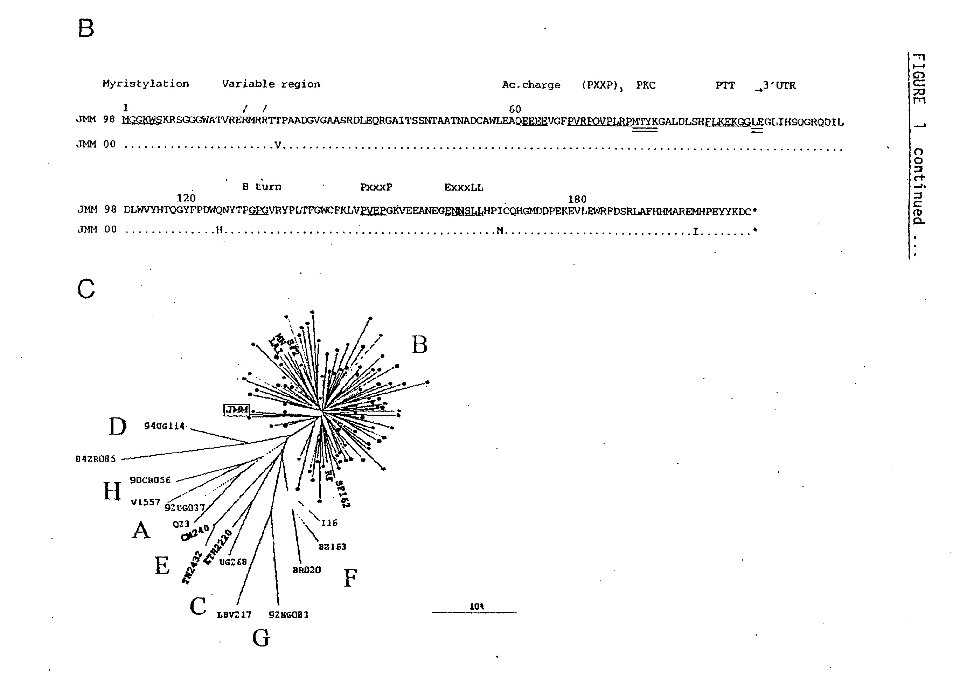

**Figure 1 shows deduced amino acid sequences of gp120, Nef and phylogenetic classification of the HIV-1 virus isolated from the LTNP donor**
   (A) Alignment of deduced amino acid sequences of HIV-1 virus isolated from the LTNP donor. (B) gp120 amino acid sequences were numbered according to the HBX2 viral reference strain. V3 amino acids for NSI M-tropic phenotype are indicated by arrows. Deduced Nef amino acid sequences from the LTNP donor HIV-1 virus. (C) Nef sequences were obtained from proviral DNA from donor samples taken in 1998 and 2000 (JMM98 and JMM00). The location is indicated of the predicted motif for them yristoylization signal, variable region polymorphism sequence, acidic charged region, (PxxP) repeat sequences, putative phosphorylation site (PKC), polypurine tract (PPT), 5' border of the 3'UTR, beta turn (GPG), and ExxxLL (for CD4-Nef-mediated endosytosis). Phylogenetic classification of the viral isolate from the LTNP donor. The C2-V3-C3 region of LTNP viral sequences was compared with 73 Spanish isolates and reference sequences from several HIV-1 subtypes using the Neighbor-Joing method. Reference B strains (LAI, MN, SF-2, SF-162 and RF) are labelled.

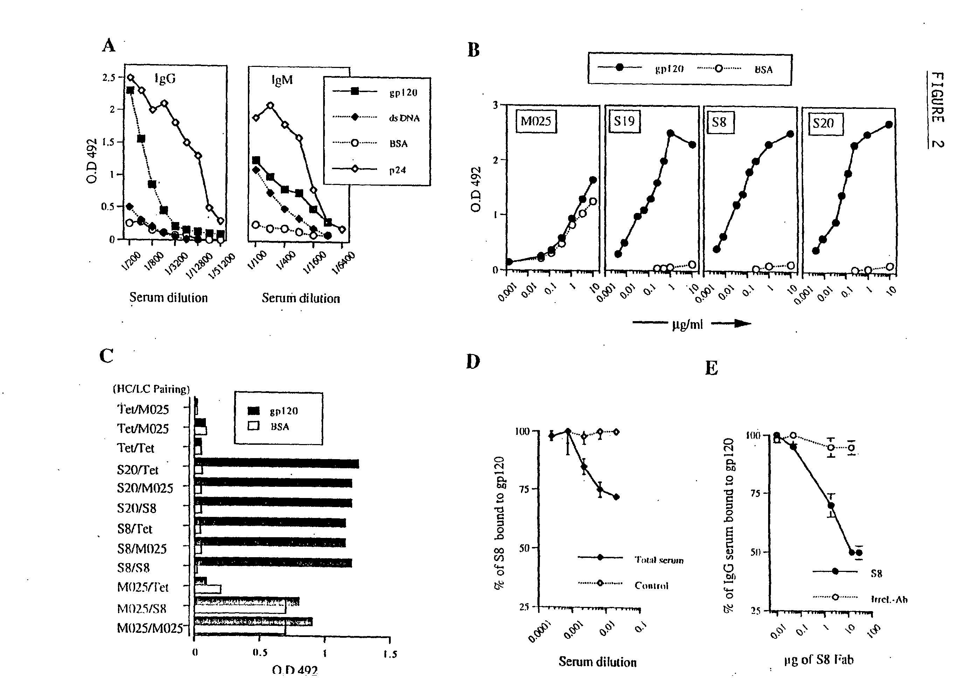

**Figure 2 shows binding properties of LTNP donor serum and anti-gp120 Fab**
   Binding properties of LTNP donor serum IgG (A, left) and IgM (right) to recombinant gp120 III-B, p24, BSA and dsDNA, tested in ELISA. (B) gp120 and BSA binding of donor-derived polyreactive IgM Fab (M025) and high affinity IgG Fabs S8, S19 and S20. (C) Light chain shuffling between polyreactive and specific anti-gp120 Fabs; heavy and light chains from IgM M025, IgG S8 or S20, and an irrelevant Fab against tetanus toxoid (Tet) were combined and the resulting Fab HC/LC pairs tested in ELISA for binding to gp120 and BSA. (D)
   Antigen binding competition between donor serum and Fab S8; Fab S8 (0.05 µg/ml) binding to gp120 III-B (2 µg/ml) was tested in ELISA in the presence of dilutions of total donor serum or human HIV-1-seronegative serum as a control; Fab S8 binding was developed using a PO-conjugated anti-histidine antibody. (E) Donor serum (1/200) was tested for gp120 III-B binding in the presence of Fab S8 (0.01-30 µg/ml) or the irrelevant Fab P1; IgG serum binding was developed using a PO-conjugated anti-human IgG Fc.

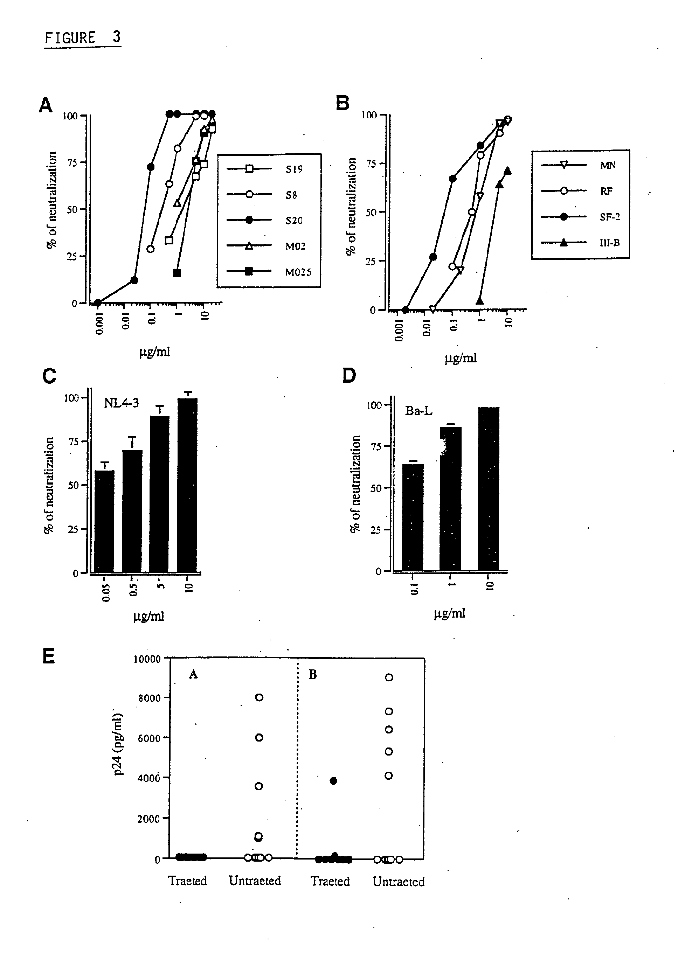

**Figure 3 shows HIV-1 neutralisation by human Fab**
   (A) Neutralisation of the HIV-1 MN strain by IgM Fabs M02 and M025 and IgG Fabs S8, S19 and S20 determined by plaque assay (NPA) in MT-4 cells. (B) Neutralisation of the T cell-adapted strains LAI, MN, RF and SF-2 by Fab S8 using the infectivity reduction assay (IRA) in MT-2 cells. (C) Neutralisation capacity of Fab S8 determined by quantification of p24 after PBMC infection with X4 (NL4-3) and (D) R5 HIV-1 strain (Bal). (E) Fab S8 *in vivo* neutralising activity of R5 (Bal) HIV-1 infection in human PBMC-reconstituted SCID mice. SCID mice grafted with adult human PBMC (SCID-hu-PBMC) s ensitive to HIV-1 infection were infected 2 weeks after reconstitution with cell-free HIV-1 Bal stocks containing 100 TCID₅₀. Mice were injected i.p. with purified Fab S8 (300 µg/mouse; treated group) or PBS alone (untreated group). Peritoneal cells were recovered after 15 days and co-cultured with PHA-activated PBMC from HIV- 1 -seronegative individuals. Co-cultures were monitored in ELISA for HIV-1 core antigen in supernatant at days 7 (left) and 14 (right), and were considered positive when p24 was >30 ng/ml.
**Figure 4 shows gp120 binding of 58 Fab in the presence of sCD4**
   Several dilutions of purified Fab S8 were tested in ELISA for binding to gp120 III-B (2 µg/ml) alone, or which had been pre-incubated with a five-fold molar excess of sCD4. Similar results were obtained using Fab S20.
**Figure 5 shows inhibition by mimopetides of Fab S8 binding to gp120**
   Fab S8 binding to gp120 was tested in ELISA in the presence of peptides derived from peptide library panning, 12R1 (A), 12R4 (B), 12R9 (D), an irrelevant peptide (C), and an HIV-1 peptide corresponding to gp120 amino acid sequence 428-439 (E).
**Figure 6 shows gp120 binding by Fab S8 and S20 HCDR3 mutants**
   Arg95 from Fab S8 (A) and S20 (B) HCDR3 was replaced by the amino acids indicated in single letter code. Binding to gp120 and BSA by these Fab mutants, as well as by the unmutated forms and the related polyreactive Fab M025, was then tested in ELISA.
**Figure 7 shows molecular model for the Fab S8 gp120 epitope**
   In A, Top: Reconstruction of the gp120 trimer model reproduction proposed by Kwong *et al.;* the bound CD4 in shown in gold. The gp120 surface is coloured by domain; inner domain in yellow (amino acids 90-117, 208-255, 474-492), bridging sheet domain in violet (amino acids 118-207, 422-439) and outer domain in red (amino acids 256-396, 410-421, 440-473). The white ball corresponds to the C-alpha of gp120 residue 299, and helps to visualise the V3 loop that is missing in the gp120 core structure. Bottom: Ball-and-stick representation of the proposed Fab S8 conformational epitope. This region mimics the sequence of the linear peptide mimotopes derived from the phage display libraries; this region is well conserved in most gp120 sequences. The figure also shows a saline bridge between Glu381 and Lys207, for which a key role has been suggested in the interdomain relationship. Arg 419, which forms a strong bond with Fab 17b , is also indicated. The position of the Fab S8 conformational epitope overlaps at least two of the residues that form part of the CD4i epitope (Arg419 and Gln422); it is clearly different from the gp120 CD4 binding site, and also differs from the well characterised V3 region.
**Figure 8 shows the nucleic acid and amino acid sequences for the S8, S19 and S20 light chains.**

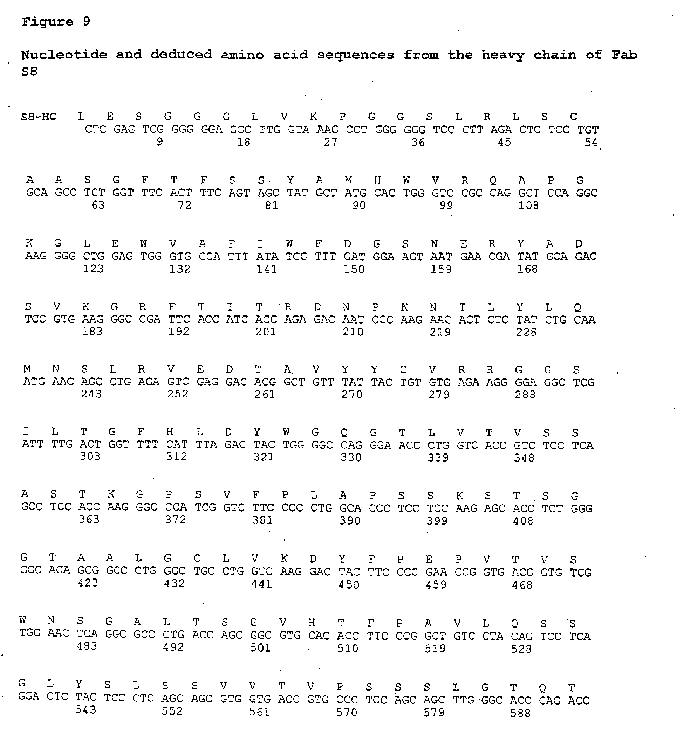

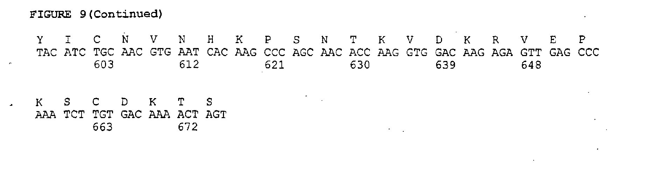

**Figure 9 shows the nucleic acid and amino acid sequences for the S8 heavy chain.**

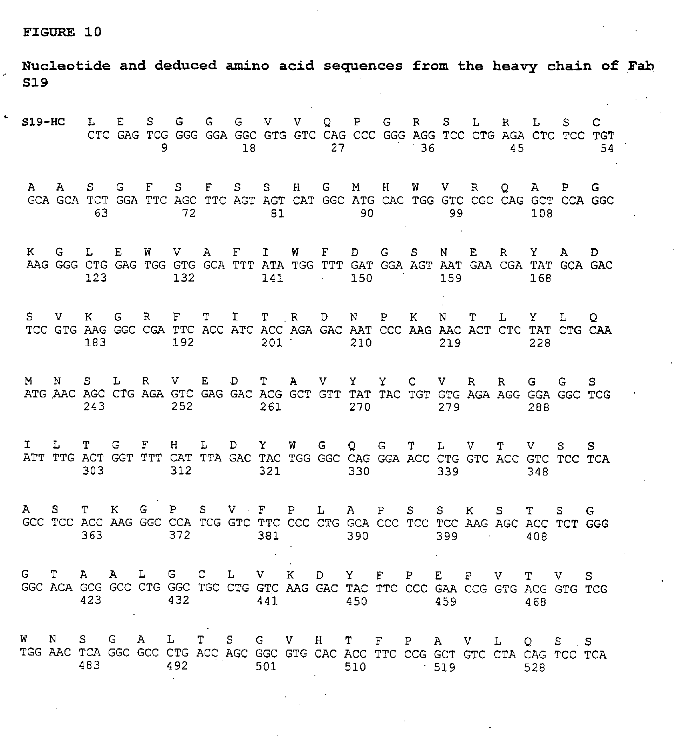

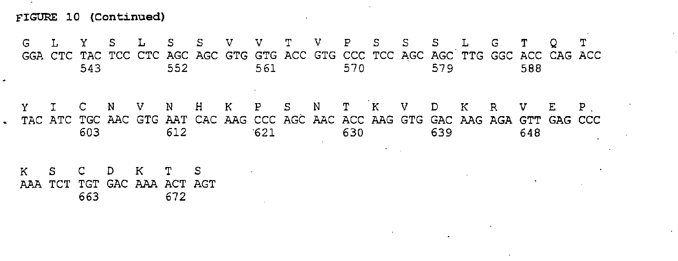

**Figure 10 shows the nucleic acid and amino acid sequences for the S19 heavy chain.**

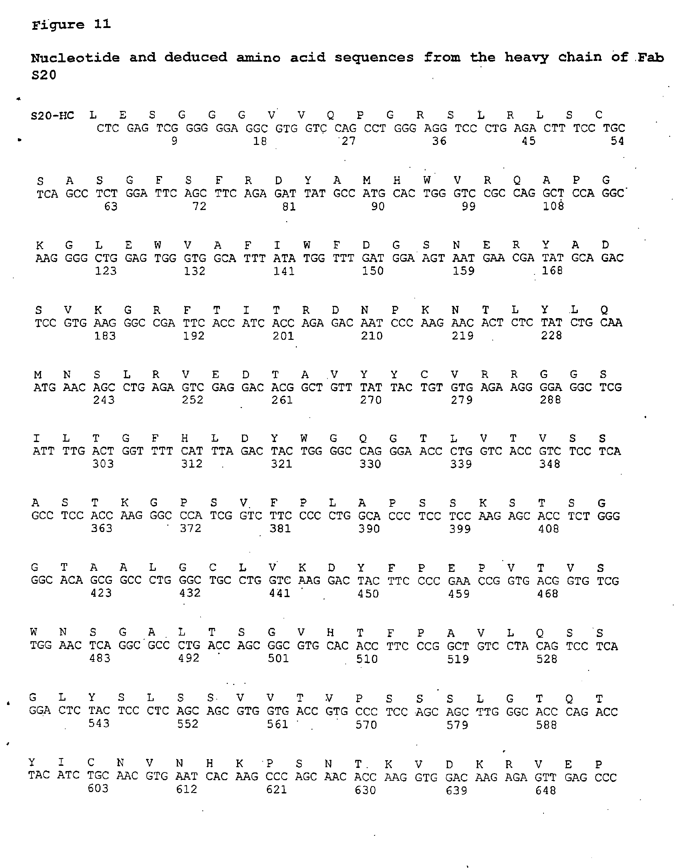

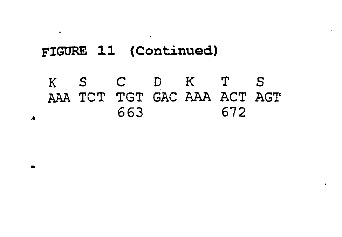

**Figure 11 shows the nucleic acid and amino acid sequences for the S20 heavy chain.**

### EXAMPLE

### MATERIALS AND METHODS

### Long-term asymptomatic HIV-1 seropositive donor

HIV-1 seroposivity from patient JMM was detected in 1985. This patient has never treated with antiretroviral agents and has maintained (>15 yr) an asymptomatic state with absolute CD4⁺ c ounts > 800-950/mm³ and 1 ow viral load, as measured periodically in PBMC (viral load (bDNA 3.0 Bayer Diagnosys) RNA viral copies/ml) over the last two years: 4367 c/ml on 05/99; 6936 c/ml on 10/99 ; 5326 c/ml on 03/00 and 7205 c/ml on 03/01).

### Allele genotype analysis of the LTNP HIV-1 donor

Genomic DNA was isolated from peripheral blood mononuclear cells (PBMC) from JMM donor using Easy DNA (Invitrogen). Up- and downstream oligonucleotide primers were used to amplify the CCR5 gene corresponding to the second extracellular region; their sequences are: 5'-primer: CCTGGCTGTCGTCCATGCTG; 3'-primer: CAAGCAGCGGCAGGACCAGC. Using this primer set, the wild-type CCR5 allele gives rise to a 245 bp polymerase chain reaction (PCR) fragment, whereas the deleted allele gives a 213 bp fragment. For each PCR reaction (100 ml), genomic DNA (1 µg) was denatured at 95°C for 5 min, amplified by 5 PCR cycles (94°C, 45 s; 55°C, 45 s; 72°C, 45 s), followed by an additional 35 cycles (94°C, 45 s; 63°C, 45 s; 72°C, 30 s). The reaction products (25 µl) were separated on a 3% Nusieve GTG agarose gel and DNA bands stained by ethidium bromide. CCR5 PCR fragments were cloned in the pCR 2.1 vector (Invitrogen) and several clones were sequenced automatically.

The CCR5 promoter region (nucleotides 59013 to 59732; GenBank Acc. No. U9526) was amplified from the genomic DNA donor by PCR as described (McDermott, *et al.* 1998) using LK84 and LK87 primers. The CCR2b gene corresponding to region 1 to 327 bp was amplified by PCR using the primers CCR2 F3 (5'-ATGCTGTCCACATCTCGTTC-3') and CCR2 R3 (5'-CCCAAAGACCCACTCATTTG-3') as described (Smith, et *al.* 1997). The 3'UTR fragment from the SDF-1β gene (nucleotides 357-1080) was PCR amplified using the primers 5-Sdf TGAGAGGGTCAGACGCCTGAGG and 3-Sdf AGTTTTGGTCCTGAGAGTCC. The PCR fragment products from genes were subcloned in pCR 2.1 (Invitrogen), sequenced automatically and compared in GenBank.

### MT-2 assay for determination of syncytium-inducing (SI) and NSI phenotypes.

The syncytium-inducing (SI) or NSI phenotype was defined by the infection of MT-2 cells as previously describe (Koot et al. 1992). Syncytia are defined as persisting large multinuclear cells with a diameter grater than 3 normal cells diameters. Virus from JMM was grown on PBMC from seronegative donors and titulate. JMM isolate, 1.3x 10³ TCID₅₀ (50% tissue culture infective doses) was mixed with MT-2 cells (10 x10⁶/ml) and in MT-2 medium (RPMI without IL2) 2 hs at 37°C. After centrifugation (10 min at 15000 rpm) cells were collected and MT-2 medium was added to complete 10 ml. Every week cells were removed and replaced with 5 x10⁶ MT-2 cell. Cultures were examined for presence of syncytia and p24 was measure from supernatants at 7, 14 and 30 days. JMM cultures were found negative for p24 and SI phenotype.

### Analysis of HIV-1 gp120 env and Nef sequences from LTNP donor virus

The gp120 *env* gene from donor JMM was derived from proviral DNA of PBMC separated by Ficoll centrifugation. The sample was amplified by nested PCR, in the first reaction with primers 128EU (5'-TTAGGCATCTCCTATGGCAGGAAGAAGCGG-3') and 129ED (5'-GTCTGGGGCATCAAACAGCTCCAGGCAAGA-3') and in the second PCR with primers 99EU (5'-AGAGCAGAAGACAGTGGC-3') and 96ED (5'-CGCACAAGACAATAA TTGTCTGGCCTGTACCGT-3'). PCRs were performed in a final volume of 50 ml, in 10 mM Tris-HCl buffer, pH 8.3 with 50 mM KC1, 0.01% gelatin, 1.5 mM MgCl₂, 100 ng of each primer and 2.5 U of Ampli-Taq polymerase (Perkin Elmer-Cetus, Norwalk, CT). Amplification conditions were 1 cycle at 94°C, 5 min, 35 cycles at 94°C, 1 min, 55°C (in the first PCR) or 58°C (nested PCR), 1 min, and 72°C, 2 min in the second reaction, followed by a final incubation at 72°C for 10 min. PCR products were cloned in the TA cloning vector (Invitrogen) and eight clones were sequenced automatically. The JMM C2-V3-C3 region was compared with sequences of the C2-V3-C3 fragment of the *env* gene from several Spanish samples amplified by a nested PCR, as described (Casado, *et al.,* 2000a).

For nucleotide data analysis, reference strains from subtypes A to H were downloaded from the Los Alamos data base (http://hiv-web.lan.gov). Nucleotide sequences were aligned with Spanish samples (Casado, *et al.,* 2000a) and the JMM sample using the CLUSTALW program (Thompson, *et al.,* 1994) and edited by hand. DNA distance matrices were calculated with the Kimura two-parameter model and used to construct a phylogenetic tree by the Neighbor-Joing method (Felsenstein, 1993). Tree robustness was evaluated by bootstrap analysis on 1000 replicas (Kumar, 1993); TreeView, version 1.5 (Page, 1996) was used to edit the phylogenetic tree. The JMM *Nef* gene was amplified by PCR from proviral DNA from donor samples taken in 1998 and 2000. Initial round of PCR was performed using primers p211 5'-TAAAGAATAGTGCTGTTAGCTTGCTC-3' and p163 5'-CTG AGGGATCTCTAG TTACCAGAG-3' followed by a second reaction with primers nef-205 5'-GCAGTAGCTGAG GGGACAGATAG-3' and nef-216 5'-GAGCTCCCAGGCTCAGATCTGGTCT-3'. Amplification conditions were 1 cycle (94°C, 5 min; 5 5°C, 30 sec; 72°C, 1 min) and 35 cycles (94°C, 30 sec; 55°C, 1 min; 72°C, 1 min) followed by a final incubation (72°C, 10 min). PCR products were sequenced automatically.

### HIV-1 donor serum and monoclonal antibodies

Serum from the LTNP donor JMM was diluted in PBS and tested for specificity to gp120, p24, and other antigens in ELISA. Wells were coated with gp120 III-B (2 µg/ml), gp41 (2 µg/ml), p24 (2 µg/ml), 3% BSA (Sigma), ssDNA (4 µg/ml), OVA (2 µg/ml), or hGH (human growth hormone, 2 µg/ml), washed, and blocked. Donor serum dilutions were incubated with antigens and developed using peroxidase (PO)-conjugated mouse anti-human IgM and IgG₁ mAb (Pharmingen, San Diego, CA). The anti-gp120 human Fabs S19, S8, and S20 and IgM Fabs M02 and M025 were obtained from the isotype IgG1, k and VH3IgM, k antibody phage display libraries constructed from donor JMM PBMC, as reported previously (Torán, *et al.,* 1999). For most experiments, Fabs were purified by Ni-NTA chromatography (Quiagen, Hilden, Germany).

For inhibition of S8 binding to gp120 by donor serum, wells were coated with gp120 III-B (2 µg/ml), and purified S8 Fab (0.05 µg/ml) was added in the presence of several dilutions of donor serum. Wells were washed and Fab S8 binding to gp120 developed using a PO-conjugated anti-histidine antibody and OPD (Sigma), and read at OD₄₉₂ nm. For inhibition of donor serum binding to gp120 by Fab S8, wells were coated with gp120 III-B as above. Donor serum (1/200) was added in the presence of Fab S8 (0.01-30 µg/ml) or irrelevant Fab P1 and IgG binding developed with PO-conjugated anti-human IgG Fc and OPD (Sigma).

### Measurement of the kinetic parameters of anti-gp120 Fabs by surface plasmon resonance

The kinetic binding constant of Fab to gp120 III-B was determined by surface plasmon resonance using a biosensor (BIAcore, Pharmacia Biosensor AB, Uppsala, Sweden). Ligand immobilisation and binding analyses were performed as described. Briefly, gp120 (10-30 µg/ml in 10 mM sodium acetate) was immobilised on a CM5 sensor chip (Pharmacia) through amine groups as recommended by the manufacturer. All immobilisation and interaction experiments were performed using HBS as running buffer (10 mM HEPES, 150 mM NaCl, 3.4 mM EDTA, 0.05% BIAcore surfactant P20, pH 7.4) at a constant flow rate of 5 µl/min. (20 µl/min. for the dissociation phase). Subsequently, 100 mM phosphoric acid was used to regenerate the binding surface. Kinetic analyses were performed with purified Fab, at concentrations ranging from 1 to 135 nM in HBS at 25°C. Kinetic rate constants (Kₒₙ and K_{off}) and the apparent equilibrium affinity constants (Kₐ = Kₒₙ/K_{off} and K_{d} = K_{off}/Kₒₙ) were determined using the BIAlogue Kinetic Evaluation Software (Pharmacia Biosensor). As a negative control, anti-tetanus toxoid Fabs were used.

### Light chain shuffling of Fab and Fab heavy chain CDR3 mutants

LC and HC fragments from M025, S20 and S8 heavy and light chains from IgM Fab M025, IgG Fabs S8 or S20, or an irrelevant Fab (Tet) were PCR amplified and cloned sequentially in the H Pcomb3 vector. Soluble Fabs from each resulting HC/LC pair were tested for binding to gp120 III-B and B SA in ELISA as above. Residue 95 was replaced in the heavy chain HCDR3 mutants of Fabs S8 and S20 and M025 by directed PCR mutagenesis. Soluble Fabs from each mutant were tested for binding to gp120 III-B and BSA in ELISA as above.

### HIV-1 Fab neutralisation

A neutralisation plaque assay (NPA) was established in M T-4 cells (Harada, et al., 1985) with minor modifications (Sánchez, *et al.,* 1993). Six-well plates (Costar, CA) were incubated with 1 ml of poly-L-lysine (50 µg/ml, Sigma) for 60 min at room temperature and washed three times in phosphate-buffered saline (PBS, pH 7). MT-4 cells (4 x 10⁶/well) were added and incubated 2 h, after which unbound cells were removed. Neutralisation was performed with 100 plaque-forming units (pfu) of virus and five purified Fabs (S20, S19, S8, M02 and M025) at different concentrations. The virus-antibody mixture was incubated (37°C, 3 h), slowly added to the plates and adsorbed (37°C, 90 min), after which virus was removed and 2 ml of agarose medium (0.2% SeaPlaque agarose in complete RPMI medium) were added. Plates were incubated (37°C, 5% CO₂ atmosphere), and 2 ml of agarose medium were added on day 3. Plaque production was counted with the naked eye on day 7.

Neutralisation titer was calculated according to the formula % neutralisation = (1 - p/n) X 100, where p is the amount of virus produced in presence of the corresponding Fab, *n* is the mean amount of virus produced without Fab, measured by number of plaques in cultures.

The following viruses were used in neutralisation experiments: HIV1 MN, HIV-1 RF, HIV-1 SF-2, HIV-1 N14-3 and HIV-1 Ba-L.

The infectivity reduction assay (IRA) was performed using in MT-2 cells. Virus titer of LAI, SF-2, MN and RF strains was determined in MT-2 cells and expressed as TCID₅₀ (50% tissue culture infective doses), calculated by the Spearman-Karber formula. Five 10-fold virus dilutions were mixed with different concentrations of Fabs S20, S19, S8, M02 and M025 and incubated (37°C, 1 h, 5% CO₂), then added to a 96-well microtiter plate containing 10⁵ MT-2 cells/well. Fresh medium (100 µl) was added 4 days later. Cytopathic effect (CPE), characterised by the appearance of giant m ultinuclear c ells, w as quantified on d ay 7. S ix replicate wells were made for each dilution. Neutralisation was calculated by the formula % neutralisation = (1 - p/n) x 100, where p is the mean titer in TCID₅₀/ml of virus produced in cultures incubated with the correspondent mixture and *n* is the mean titer of virus produced in cultures incubated without Fabs. Each titer point is the mean of two individual experiments.

PBMC obtained from the patient in 1996 were cocultured with HIV-1-seronegative PBMC which had been stimulated for 3 days with phytohemagglutinin (PHA). Coculture was maintained in medium with interleukin-2 (IL-2) for at least 40 days (37°C, 5% CO₂). Fresh PBMC were added each week and p24 antigen production was monitored every 7 days. Supernatant was harvested and characterised by TCID_{50%} on stimulated PBMC. Supernatant (1 ml) from the coculture (1.3 x 10³ TCID_{50%}) was inoculated in 10 x 10⁶ PBMC. After incubation (1 h, 37°C), cells were centrifugated and 10 ml of RPMI with IL-2 were added. Virus was grown and harvested. This first-passage virus stock was used to perform the IRA in PBMC. Four 4-fold viral stock dilutions were incubated with several concentrations of Fab S8 (1 h, 37°C) and added to 2 x 10⁵ PBMC. Medium with IL-2 was changed twice a week. After 14 days, the p24 assay was performed and TCID_{50%} calculated. Neutralisation titer was calculated according to the formula given above.

For Fab S8 neutralisation of HIV-1 viruses Bal and NL4-3, PBMC from an uninfected donor were activated with PHA (10 ng/ml, 48 h, 37°C, 5% CO₂); after washing, cells were incubated with Bal or NL4-3 viral stock (2 ng/10⁶ per assay, 30 min, 37°C), alone or with purified Fab (0.05 at 10 µg/ml) in complete RPMI 1640 medium containing rhIL-2 (10 ng/ml). Excess virus and Fab were removed by washing, and PBMC incubated in complete RPMI 1640 at 37°C. Every two days after infection, half the culture supernatant (500 µl) was removed and replaced with fresh medium containing rhIL-2 and Fabs at the above concentration. Cell-free supernatants were tested for HIV-1 p24 antigen on day 7 using a commercial ELISA test (Coulter, Miami, FL). The percentage of neutralisation was calculated as the ratio b etween p 24 levels for test samples a lone or with Fabs. Irrelevant HmFab P1 (10 µg/ml) was used as a negative control.

### SCID mouse reconstitution and HIV-1 viral challenge

CB.17 SCID/SCID mice were bred and maintained under specific pathogen-free conditions in the Centro Nacional de Biotecnologia animal facility. Eight- to 10-week-old non-leaky phenotype mice were reconstituted by i.p. injection of 20 x 10⁶ freshly isolated normal human PBMC. Four hours before viral challenge and for the next two days, mice were injected i.p. with purified Fab S8 (100 µg/mouse) in PBS or with PBS alone. Mice were infected 2 weeks after PBMC reconstitution by i.p. injection of 0.5 ml of diluted cell-free HIV-1 Bal stocks containing 100 TCID₅₀. Two weeks after viral challenge, mice were killed by cervical dislocation and peritoneal cells obtained by washing with ice cold PBS. Cells (1 x 10⁶) were incubated with phytohemagglutinin (PHA)-activated PBMC (1 x 10⁶) from HIV-1-seronegative donors, in RPMI 1640 with 10% heat-inactivated FCS and recombinant IL-2 (10 ng/ml). Co-cultures were monitored by ELISA for HIV-1 core antigen in supernatant on days 7 and 14, and were considered positive when p24 was >30 ng/ml.

### Selection of Fab S8-binding peptides from peptide phage display libraries

Peptide phage display libraries Ph.D.-7, Ph.D.-C7C and Ph.D.-12 were purchased from New England BioLabs. For parming selection of peptide-binding phages, microtiter wells were coated (4°C, overnight) with 50 µl of purified Fab S8 (1 µg/ml in PBS), washed three times with water, and blocked with 3% BSA in PBS (37°C, 1 h). To reduce non-specific phage peptide binding, peptide phage display libraries were previously incubated with human F(ab)₂ with 0.5% BSA (37°C, 1 h). For each selection round, wells were filled with 50 µl of the corresponding library (2 x 10¹¹ pfu) and incubated (37°C, 2 h), washed vigorously several times with PBST (PBS/0.05% Tween 20), binding phages eluted with glycine-HCl, pH 2.5 and BSA (1 mg/ml), and rapidly neutralised with 1 M Tris-HC1. Samples of eluted phages were titrated according to manufacturer's instructions. Eluted phages were amplified in E. *coli* ER2537, PEG concentrated, and used for the next selection round. For identification of phage peptide binding clones, independent blue phage plaques from selection rounds were randomly picked, amplified, and their DNA prepared and sequenced automatically. Additional panning rounds were performed under similar conditions. As a control, phagemid binding background was titrated in the last rounds of panning as above, except that Fab S8 was omitted.

### Peptide inhibition of gp120-Fab binding

Peptides from selected phage clones were synthesised by Isogen (Maarssen, The Netherlands). For binding competition experiments, purified S8 Fab (1 µg/ml) was incubated (4°C, 4 h) with several dilutions of the corresponding peptide before addition to gp120 III-B (2 µg/ml)-coated microtiter wells. Wells were incubated (37°C, 1 h), washed with PBST, and Fab S8 binding to gp120 was developed with PO-conjugated goat anti-human F(ab)'₂ (Pierce) and OPD.

### Molecular modelling

Structural and solvent accessibility were analysed with WhatIf (Vriend, 1990) and Grasp (Nicholls, *et al.,* 1991). Figures were rendered with Insight II (v. 98.0, Molecular Simulations). T he structures used for analysis were obtained from the Protein D ata B ank (PDB) data base (http://www.rcsb.org/pdb/). The structures used for analysis, 1GC1, 1G9N and 1G9M (Kwong, *et al.,* 2001; Kwong, *et al.,* 1998), were obtained from the Protein Data Bank (http://www.rcsb.org/pdb/). The trimer model was generated by manual fitting based on the model proposed by Kwong *et al.* (Kwong, *et al.,* 2000). For variability gp120 surface mapping, gp120 sequence alignment was obtained from the Pfam database (Bateman *et al.* 2000). For more details about alignment and the set of gp120 HIV-1 sequence used, see web page information (below). Figure was draw with Insight II Version 98.0 Molecular Modelling System. Additional figures and information are available at: "http://www.cnb.uam.es/∼cnbprot/S20/".

### RESULTS

### Allele genotype and phylogenetic analysis of the LTNP donor and HIV-1 virus

The donor (JMM) analysed in this study is an untreated HIV-1 seropositive LTNP individual (>15 yr HIV-1 infection at study); he has maintained normal CD4 counts and a low viral load to the present (see Methods). To determine whether chemokine receptors or chemokine ligand gene alleles associated with AIDS delay are present, extensive DNA genotype analysis was performed, including CCR5 and CCR2b chemokine receptors, CCR5 promoter, and the 3'UTR SDF-1β chemokine. The analysis reveals non-mutant alleles for CCR5, CCR2 chemokine receptors, CCR5 gene promoter and the 3 'UTR of SDF-1β, indicating that the LTNP donor phenotype is not due to known genetic factors associated with a delay in AIDS development.

The inventors next identified the HIV-1 viral strain in this individual. Donor PBMC were obtained and used to isolate proviral DNA by PCR. The *env* gene was amplified from proviral DNA and cloned. gp120 was fully sequenced from several independent clones; the derived amino acid sequences are shown (Fig. 1A). gp120 sequence variation of 3.02% was found among the clones analysed. This level of genetic variation is similar to that found in patients (Myers, *et al.,* 1992). The maximum distance between quasi-species members was 6.07% (between clones 50-10 and 50-9), and the minimum distance was 0.2% (between clones 50-3 and 50-1). Two of the clones that displayed deletions in the V1 loop also had a glycosylation site at position 299, like that found at position 289 in the LAI clone HXB2. A new glycosylation site, not present in HXB2, was found at position 409 in three members of the quasi-species (Fig. 1 A). Primary isolate virus from the LTNP donor was obtained by passage on PBMC from healthy seronegative individuals. The virus failed to grow and form syncytia in MT-2 cells. In addition, analysis of gp120 V3 region from several donor viral clones isolated show NSI/M-tropic amino acid (S³⁰⁶ and E³²⁰) markers (De Jong, *et* al., 1992; Connor *et al* 1997; Shankarappa, *et al.,* 1999), concurring with the NSI viral phenotype observed.

The LTNP HIV-1 *Nef* gene was amplified from donor proviral DNA at two study points and fully sequenced; derived amino acid sequences are shown (Fig. 1 B). Low (less than 1 %) variation between samples was observed, and no deletions or frameshifts resulting in premature stop codons of *Nef* were found. In addition, all predicted functional Nef motifs were conserved in LTNP virus. To study the phylogenetic classification of the LTNP isolate, we compared its gp120 sequences with those of 73 Spanish isolates (Casado, *et al.,* 2000) and reference sequences from several HIV-1 subtypes (Myers, *et al.,* 1992). The analysis was carried out in the gp120 C2-V3-C3 region by the Neighbor-Joing method (Felsenstein, 1993); the resulting tree is shown in Fig. 1C. The LTNP isolate was included in the B clade, along with Spanish and reference B strains (LAI, MN, SF-2, SF-162 and RF) with a high bootstrap value.

### Antibody response to HIV-1 gp120

Donor JMM serum IgM and IgG binding to gp120 and p24 was analysed in ELISA; high IgM and IgG titers to both antigens were found (Fig. 2A). We previously reported isolation of a panel of IgM and IgG₁ anti-gp120 Fabs by gp120 biopanning from two antibody isotype phage display libraries (corresponding to the IgM and IgG repertoires) constructed from this donor (Torán, *et al.,* 1990). In these experiments, we found that the IgM Fabs bind to gp120 with low affinity and react to several antigens, whereas IgG Fabs retrieved were specific, with high affinity (Kd, 2.2 x 10⁻⁹ to 9.5 x 10⁻¹⁰ M) for gp120 (Fig. 2B). Analysis of IgM Fab VH genes showed use of a variety of germ line genes, most unmutated. In contrast, all the IgG Fabs isolated were derived from a single VH3 germ line gene (DP50), showed evidence of extensive somatic mutation, and HCDR3 analysis indicated common clonal origin. The IgG Fabs nonetheless had different affinity constants for gp120 III-B, as measured in BIAcore. These affinity differences are due to amino acid changes in their FR1 and HCDR1 regions, originated by somatic mutation, which led to a 10-fold increase in affinity for gp120 (Torán, *et al.,* 2001). The relationship between these two isotypes from this donor was shown by VH and HCDR3 analysis of IgM and IgG Fabs coded by the VH DP50 gene, and suggest that one IgG Fab, S8, arose from IgM Fab M025.

Although the VH from these two Fabs have common somatic mutations and differ mainly in the HCDR3 regions, light chain (LC) molecular analysis of Fabs S8 and M025 showed different HC/LC pairings. The original B cell HC/LC pairing can be lost using the combinatorial approach of the donor k LC repertoire with the different HC (IgM and IgG) repertoires; nonetheless, it is known that most antibodies retain their specificity when a particular HC is paired with different LC (Collet, *et al.,* 1992). We thus analysed the binding properties of each HC/LC pair in both Fabs by interchanging the LC from Fabs S8 and M025, and tested gp120 binding by these combinations in ELISA. The results show that Fab S8 gp120 specificity was unaffected by pairing its HC with LC from M025 or from an irrelevant Fab (Tet) (Fig. 2C). Moreover, combination of Fab M025 HC with Fab S8 LC did not modify the polyreactivity observed for M025. All data thus indicate that the LC have a minor role in antigen recognition, and that antigen binding specificity differences are governed by the heavy chains in these Fab.

Fab S8 specificity representation in the donor antibody repertoire was demonstrated in ELISA by gp120-Fab binding inhibition by total donor serum collected at same time as the PBL used to construct the combinatorial libraries (Fig. 2D). In addition, gp120 binding of serum IgG was inhibited by the S8 Fab (Fig. 2E). This shows that gp120 antibody specificities selected using the antibody phage display approach are represented in the donor humoral response to HIV-1 gp120.

Taken together the data indicate that, as for other humoral responses, HIV-1 elicited a polyreactive primary IgM response and a high affinity IgG response to gp120 in this LTNP. Maturation of the primary antibody response included accumulation of VH and CDR3 somatic mutation and isotype switching, resulting in a specificity change (from polyreactive to specific antibodies) associated with affinity increase (100-fold) for gp120, as illustrated by the IgG Fab.

### Kinetic parameters of the anti-gp120 Fabs measured by surface plasmon resonance

The results for each Fab are shown below:

| **Fab** | **K**_{**on**}**(CM**^{**-1**}**s**^{**-1**}**)** | **K**_{**off**}**(s**^{**-1**}**)** | **Ka(M**^{**-1**}**)** | **Kd(M)** |
|---|---|---|---|---|
| S19 | 4.8 104 | 4.0 10⁻⁴ | 1.2 10⁸ | 8.3 10⁻⁹ |
| S8 | 5.4 10⁴ | 1.2 10⁻⁴ | 4.5 10⁸ | 2.2 10⁻⁹ |
| S20 | 19.010⁴ | 1.8 10⁻⁴ | 10.5 108 | 9.5 10⁻¹⁰ |

### HIV-1 neutralisation by the human anti-gp120 Fabs

The HIV-1 (MN strain) neutralisation capacity of purified Fabs was determined by NPA in MT-4 cells (Fig. 3A). Distinct patterns were observed in this assay, depending on the Fab concentration required for 100% neutralisation. Fab S20 reached 100% neutralisation at the lowest concentration (1 µg/ml). Fab S8 showed 100% neutralisation at 10 µg/ml; for Fab S19, 92% neutralisation was observed at 20 µg/ml. The IgM Fabs (M02 and M025) derived from the same DP50 germ line gene as Fabs S8, S19 and S20 had similar neutralisation patterns, with 90% neutralisation at 10 µg/ml. At 20 µg/ml, all Fabs display 90% neutralisation.

Fab S8 was selected to study neutralisation against different T cell-adapted (TCA) strains (LAI, MN, RF and SF-2), using the IRA in MT-2 cells (Fig. 3B). Several Fab S8 concentrations were used to neutralise five 10-fold dilutions of each virus. Fab S8 neutralised all TCA strains tested, although the neutralising concentration varied among strains. At 1 µg/ml of Fab S8, only SF-2, MN and RF were 50% neutralised. At 10 µg/ml, neutralisation values for MN, SF-2 and RF were greater than 90%, whereas the HIV-1 LAI strain was poorly n eutralised at the s ame F ab c oncentration. The donor virus isolate was neutralised using an IRA assay in PBMC; at 25 µg/ml of Fab, 32% neutralisation was observed. Fab S8 neutralisation capacity was also determined by quantification of p24 after PBMC infection with the HIV-1 X4 (NL4-3) and R5 (Bal) strains (Fig. 3C, D). In this assay, 50% neutralisation of NL4-3 and Bal was observed with less than 0.1 µg/ml of Fab S8.

They extended the *in vitro* neutralisation results of Fab S8 to *in vivo* R5 (Bal) strain infection in SCID mice reconstituted with human PBMC. Human PBL-grafted SCID mice (SCID-hu-PBMC) are sensitive to HIV-1 infection; they consequently undergo loss of human CD4+ T lymphocytes, making them suitable to study the mechanisms of HIV-1 pathogenesis and potential therapeutic treatments (Mosier, 1996). SCID-hu-PBMC mice were injected with 100 µg purified Fab S8 or PBS before infection with 100 TCID₅₀ of HIV-1 Bal. After viral infection, two additional doses of Fab S8 were administered. Mice were sacrificed after 15 days, peritoneal cells recovered and co-cultured with PHA-activated human PBMC. HIV-1 p24 from co-culture supernatants was measured on days 7 and 14 using a commercial kit. p24 was undetectable in 100% of Fab-treated mice on day 7, and in seven of eight on day 14, while 41% of control mice showed high p24 levels (Fig. 3E). The data indicate that Fab S8 also has *in vivo* neutralising activity for the M-tropic R5 HIV-1 Bal strain in SCID-hu-PBMC mice.

### Characterisation of HIV-1 gp120 epitope recognised by neutralising IgG Fabs

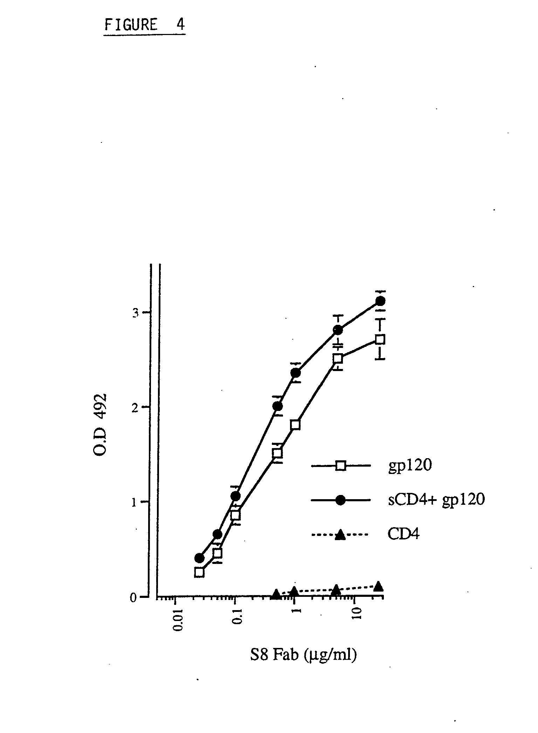

Using phage display, other groups have reported the isolation of recombinant Fabs directed to the gp120-CD4 binding site. Competition for Fab S8-gp120 binding by soluble CD4 (sCD4) does not reduce Fab binding to antigen (not shown). Nonetheless, preincubation of gp120 with CD4 shows a 30% increase in Fab binding to gp120 as measured by ELISA (Fig. 4). These results indicate that the gp120 epitope recognised by the Fab is probably better exposed following interaction with CD4. Nonetheless, this increase in Fab-gp120 binding is less pronounced than that reported for CD4i antibodies (17b and 48d), which only bind to gp120 in the presence of sCD4 (Sullivan, *et al.,* 1998; Thali, *et al.,* 1993).

Several approaches were used to characterise the gp120 epitope that recognises the IgG Fabs isolated from donor JMM. A collection of overlapping 20-mer peptides corresponding to the gp120 III-B (LAI) amino acid sequence was prepared on cellulose, and epitope mapping performed using purified Fab S8 and goat anti-human antibody. A non-unique peptide motif corresponding to the primary sequence was identified, suggesting a conformational gp120 epitope (not shown). We then used a set of peptide phage display libraries to map the Fab-gp120 epitope. Phage clones binding to Fab S8 were identified by reacting three different peptide phage libraries, Ph.D.-C12 (12 amino acids), Ph.D.-C7 (seven amino acids) and Ph.D.-C7C (seven cycled amino acids), with the Fab. After four panning selection rounds, significant phage enrichment was found in libraries Ph.D.-C12 and Ph.D.-C7C. DNA from eluted individual phage clones, corresponding to each selection round, were sequenced to deduce peptide amino acid sequence (Table 1).

Using the Ph.D.-C12 library, eight independent peptide sequences were identified from the last selection round. Eleven phage clones analysed were unique and shared the same nucleotide and peptide sequence displayed by clone c124R4 (LLADTTHHRPWT). Peptide phage clones c124R9 (GIQLANPPRLYG) and c124R1 (FLQPPDFSHLPP) were found four and two times, respectively, whereas the other phage clones were found once each. In addition, peptide phage clones c124R4 and c124R9 were also found within the phage clones analysed from the second and third selection rounds.

All independent clones retrieved from the last selection round of the Ph.D.-C7C library had distinct peptide sequences, and a non-consensus motif was identified. After four rounds of Fab S8 selection, library Ph.D.-C7 rendered phages displaying different peptide sequences, although clone c72R4 (SAMEAPP) showed a similar sequence motif to clone c124R9 from library Ph.D.-C12.

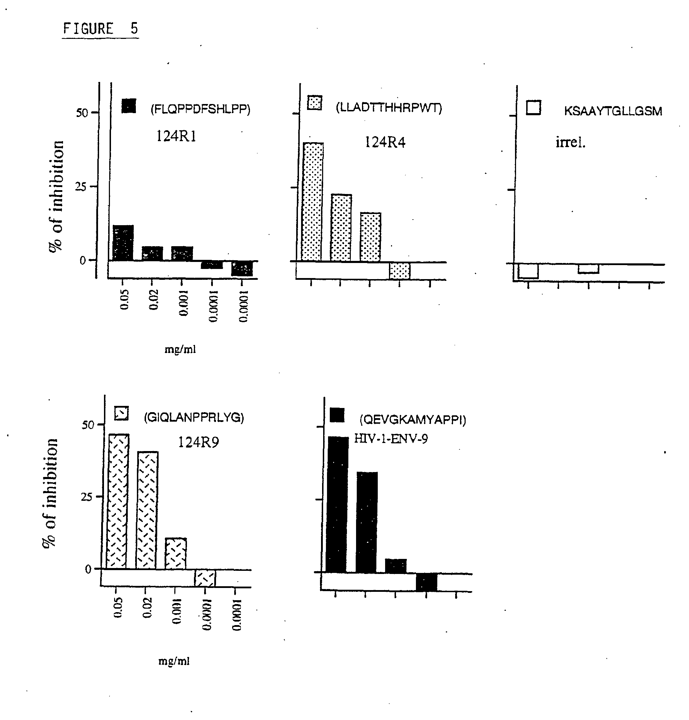

Although no evident consensus amino acid motif was found in all peptide phage clones, most peptides had two consecutive proline residues. Peptides from clones c124R9 (GIQLANP PRLYG), c124R4 (LLADTTHHRPWT), c124R1 (FLQPPDFSHLPP) and peptide ENV-9 (QEVGKAMYAPPI) corresponding to amino acid residues 428-439 from the gp120 with which 124R9 peptide and c72R4 can be aligned, were synthesised and tested in ELISA for inhibition of Fab S8 binding to gp120. Peptides 124R4 and 124R9 showed 50% inhibition of Fab S 8-gp 120 binding at 50 µg/ml, whereas p eptide 124R1 showed only 15% at a similar concentration (Fig. 5). Moreover, peptide ENV-9 showed 50% inhibition of Fab S8-gp120 binding. An unrelated negative control peptide showed no Fab S8-gp120 binding inhibition activity.

### Structural analysis of the conformational Fab-gp120 epitope interactions

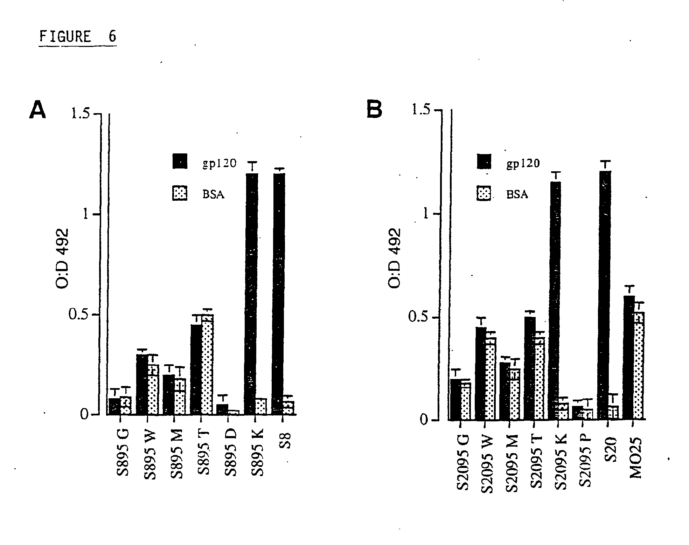

The importance of the HCDR3 region in Ab-Ag binding has been reported (Morea, *et al.,* 1997); amino acid residues from this region are frequently responsible for Ab-Ag interactions. For the HIV-1 neutralising IgG Fabs S 8, S 19 and S20, a molecular model for their heavy chains suggest a key role for the HCDR3 loop in contacting antigen (Torán, *et al.,* 1999). IgG from the high affinity neutralizing Fabs S8, S19 and S20 have a charged amino acid residue (Arg) at position 95 in the HCDR3 loop, whereas the polyreactive IgM Fab M025 has a Thr at this position, suggesting that the presence of Arg95 in HCDR3 has a fundamental role in antigen specificity and binding. The key role of this residue in IgG Fabs was analysed by generation of Arg95 Fab mutants and determination of their gp120 binding properties in ELISA. Results show that replacement of Arg95 by Asp, Pro or Gly abolished Fab binding to gp120; replacement by an amino acid of similar charge (Lys) in mutant Fabs S895K or S2095K showed no change in gp120 specificity (Fig. 6). In contrast, Fabs in which Arg95 was replaced b y Trp, Met or Thr (the last is the equivalent residue in Fab M025) showed reduced binding and change in gp120 specificity.

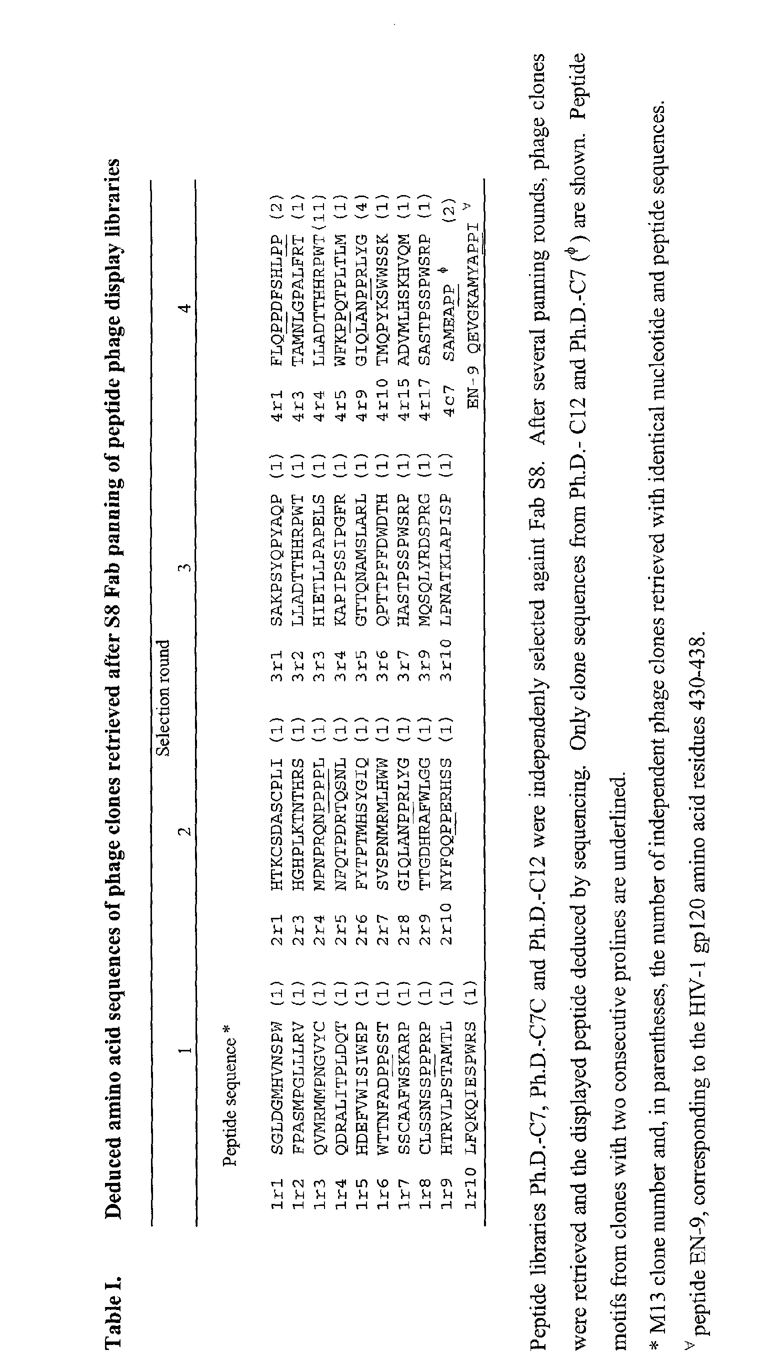

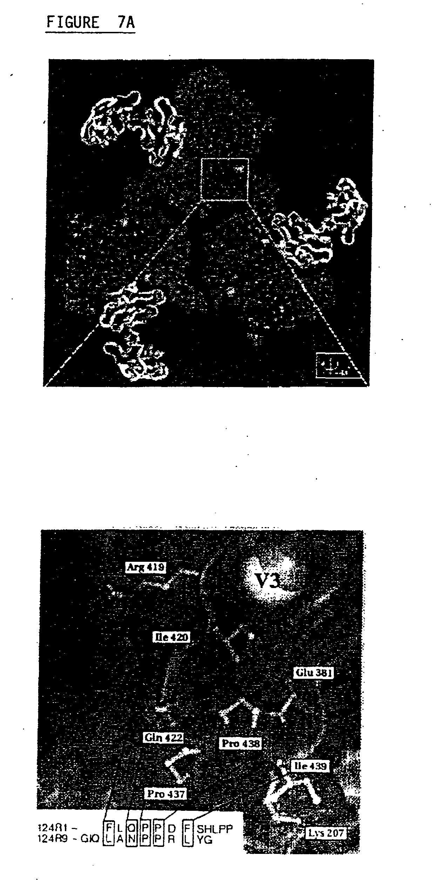

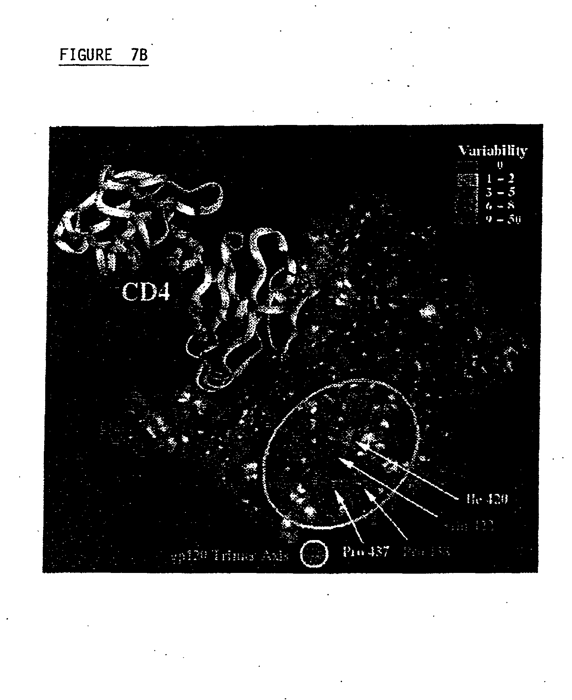

To analyse the gp120 epitope structure recognised by Fab S8, we compared peptide sequences derived from peptide phages with the amino acid sequence of several HIV-1 envelopes, including gp120 from donor JMM using Clustal W (Thompson, *et al.,* 1994). Partial similarity was found around the two consecutive prolines in some peptides and gp120, probably reflecting the random nature of phage peptide display, in which specific amino acid residues can mimic the true antigen epitope. Based on the gp120 core structure, we searched manually for conformational surface sequences corresponding to Fab S8-binding peptides. Only peptides 124R9 (GIQLANPPRLYG) and 124R1 (FLQPPDFSHLPP) result in a conformational epitope, and align at residues 420-422 and 437-439 with two gp120 regions (Fig. 7 A), thus sharing amino acids with the gp120-CCR5 binding region (Rizzuto and Sodroski, 2000). Amino acid variability of Fab S8 epitope was analysed by alignment of a large number of HIV-1 gp120 sequences (including sequences from M and T tropic virus) from Pfam (Protein families database; Bateman *et al.* 2000). Variability was calculated from a 99% non-redundant gp120 alignment (without gp120 fragments) using the McLachlan matrix (McLachlan, 1971), and mapped over the gp120 core structure surface (Kwong, *et al.,* 1998). Amino acids Ile420, Gln422, Pro437 and Pro438, which compose the S8 Fab epitope, showed low variability, indicating a high degree of conservation in most HIV-1 viruses (Fig. 7b).

Considering these data and the mutagenesis experiment results for Fab S8 Arg95, the inventors searched for charged amino acid residues (Asp or Glu) near the putative gp120 Fab epitope and found only Glu381 as a candidate for establishing an electrostatic interaction with Arg95 in the HCDR3 loop of Fab. In addition, we found that Glu381 was conserved in HIV-1 viruses (see web page). Previous observations indicate that Glu381 and Lys207 form a salt bridge between the inner and outer domains of C D4-bound gp120 (Rizzuto and S odroski, 2000). Furthermore, changes in Glu381 or Lys207 abrogate CCR5 binding, demonstrating the importance of these residues in gp120 interdomain relationships and correceptor binding. A hypothetical interaction between Arg95 from the Fab S8 HCDR3 loop and Glu381 in gp120 could thus break an inaccessible high energy saline bond (Hendsch and Tidor, 1994; Sindelar, *et al.,* 1998), resulting in a change in gp120 inner-outer interdomain relationships.

### DISCUSSION

Among HIV-1 infected persons, long-term non-progressor (LTNP) comprise a reduced group of infected individuals who tolerate infection without immune suppression for >10 years in the absence of antiretroviral therapy. These individuals manifest a potent humoral response able to neutralize *in vitro* several HIV-1 isolates, providing an opportunity to study the role of the humoral response developed as consequence of natural HIV-1 infection. Although the role of the antibodies in protective immunity against HIV-1 is not known, data indicate that discontinuous envelope epitopes, rather than linear epitopes, may be the targets of efficient neutralizing antibodies. Conformational epitope-directed antibodies are the majority of anti-HIV-1 1 glycoprotein antibodies in HIV-1-infected individuals. This type of antibody has been not detected in vaccinated volunteers, in whom immunogens elicit antibodies to linear epitopes with diverse specificities, which neutralise TCLA viruses (Mascola, *et al.,* 1996), but not primary isolates (Beddows, *et al.,* 1999; Loomis, *et al.,* 1995). Specific high affinity human antibodies against conformational epitopes can be obtained using the antibody phage display approach, which also permits analysis of the human antibody repertoire developed as a consequence of natural infection. We previously constructed two antibody phage display isotype (IgM and IgG) libraries from an HIV-1-infected LTNP (>15 yr) donor (Torán, *et al.,* 1999). From these libraries, several Fabs were selected by gp120 antigen panning; IgG Fabs retrieved were of high affinity and gp120-specific, whereas IgM Fabs were of low affinity and polyreactive.

Here the inventors have extended these results, have performed an exhaustive analysis of chemokine genes associated with AIDS delay, and have characterised the LTNP HIV-1 virus. DNA genotyping of the donor shows no alleles related to the principal human genes reported to produce a delay in AIDS development (O'Brien and Moore, 2000), indicating that the phenotype of this LTNP donor is not due to such genetic factors. The donor virus isolate was classified as N SI, based on its phenotype in M T-2 cells. Analysis of gp120 showed non-significant sequence variation, indicating a homogeneous HIV-1 virus isolate, and that the V3 region from gp120 had M-tropic amino acid markers that correlate with the NSI phenotype observed. No defective Nef gene alleles from the LTNP virus were found. P hylogenetic classification showed that this LTNP HIV-1 isolate belongs to clade B, the predominant subtype in Spain (Casado, *et al.,* 2000a, b).

To extend the analysis of the primary (IgM) and secondary (IgG) antibodies obtained from the donor, high affinity anti-gp120 IgG Fabs were characterised extensively. IgG Fab binding properties can attributed principally to the heavy chains. Our results indicate a minor role for light chains in IgG Fab S8 and S20 binding and specificity properties; a combination of the S8 heavy chain with the light chain from a clonally related polyreactive IgM Fab (M025) or an unrelated non-specific Fab had no effect on Fab S8 gp120 binding and specificity. Although the original heavy and light chain pairing can be lost during generation of the antibody library using the combinatorial approach, we observed that IgG Fab S8 and serum from the LTNP donor were able to compete for gp120 binding. These data indicate that Fab S8 anti-gp120 specificities retrieved from the library are well represented in donor serum and are not new antibody specificities generated by the randomness of the approach (Persson, *et al.,* 1991). This also confirms the utility of the antibody display method to study the humoral immune response repertoire (Barbas, *et al.,* 1993; Ditzel, *et al.,* 1997).

Compared with IgG Fab, all IgM Fabs selected from the donor were polyreactive, with low affinity for gp120. One polyreactive IgM Fab (M025) derived from the same germline gene as that coding for IgG Fab S8 shared common VH nucleotide sequences, with amino acid changes caused by identical somatic mutations. HCDR3 similarities also suggested a relationship between these two F abs. H ere we show that the H CDR3 amino acid residue differences between these two Fabs play a significant role in Fab gp120 specificity and affinity. Results indicate that replacement of HCDR3 Arg95 by Asp, Pro or Gly abolished Fab S8 binding to gp120; moreover, Arg95 replacement by Trp, Met or Thr (this last is the native residue in Fab M025) results in gp120 binding and specificity changes. These findings concur with recent experiments using a transgenic mouse model with a limited V region but full CDR3 diversity. Results from these studies showed that HCDR3 diversity was sufficient for most antibody specificities, and that somatic mutation allows achievement of surprisingly high antibody affinities.

All DP50-derived Fabs (IgM and IgG) isolated from this donor were able to neutralise the laboratory H IV-1 s train MN. F ab S 8 n eutralisation c apacity w as also t ested u sing several methods and HIV-1 strains; this Fab neutralised X4 HIV-1 strains MN, RF, SF-2, III-B and NL4-3, as well as the R5 Bal strain. Moreover, Fab S8 neutralized M-tropic Bal infection *in vivo* in human PBMC-reconstituted SCID mice. These data indicate that anti-gp120 Fab S8 isolated from the LTNP donor is a potent *in vitro* and *in vivo* inhibitor of HIV-1 infectivity.

To further characterise Fab S8, we mapped the gp120 epitope using several methods. Previous experiments using gp120 overlapping peptides epitope suggests that Fab S8 recognises a non-linear epitope. We then used a set of random peptide phage libraries as an alternative tool to map the S8 epitope (Boots, *et al.,* 1997; Ferrer and Harrison, 1999; Ferrer, *et al.,* 1999; Schellekens, *et al.,* 1994; Scott and Smith, 1990; Yip and Ward, 1999). Most phages retrieved after panning with S8 Fab had peptides with a motif of two consecutive prolines. Peptides from the most frequently selected phages were chosen, synthesized and tested for S8-gp120 binding competition. Our results indicate that peptides 124R1, 124R9 and 124R4 showed significant inhibition of S8-gp120 binding. In addition, peptide ENV-9, corresponding to gp120 amino acid residues 428-439 and chosen for similarity to 124R9 and to 72R4 (a peptide derived from phage clone c72R4 by panning of peptide library Ph.D.-C7), also inhibited Fab S8-gp120 binding. Alignment of candidate peptides with the amino acid sequence of several HIV-1 envelopes, including donor gp120, as well as peptides 124R9 and 124R1, showed only partial similarity around the two consecutive gp120 prolines (Pro437 and Pro438). These two prolines were recently described as key residues implicated in the gp120 coreceptor binding site (Rizzuto and Sodroski, 2000).

To study the Fab S8 epitope in detail, we used molecular modelling to search for conformational gp120 core structure-based surface sequences that correspond to the Fab S8 binding peptides. Our model predicts that peptides 124R9 and 124R1 can result in a conformational epitope that aligns with two gp120 regions at residues 420-422 and 437-439. Amino acids from these regions (Ile420, Lys421, Gln422, Pro438) have been described as components of the gp120-CCR5 binding region. Mutagenesis experiments indicate that modification of these residues, as well as of Gly441, had specific consequences on CCR5 binding, with little effect on binding to CD4 (Rizzuto and Sodroski, 2000); monoclonal antibodies 17b and 4 8d are also reported to bind amino acids in this region (Thali, *et al.,* 1993). These Ab bind gp120 and neutralize HIV-1 efficiently (Salzwedel, *et al.,* 2000) only in the presence of CD4, defining an inducible CD4 (CD4i) epitope on gp120 (Sullivan, *et al.,* 1998; Thali, *et al.,* 1993).

The results suggest that certain gp120 amino acids recognized by Fab S8 are shared with those recognized by mAb 17b, although compared to 17b, we observed little CD4 dependence on Fab S8 binding to gp120 (soluble CD4 previously bound to gp120 increased Fab-gp120 binding by only 30%). The Fab S8 epitope is thus defined as CD4i-like (CD4il). Differences between epitopes 17b-CD4i (Sullivan, *et al.,* 1998) and S8-CD4il may be due to a) the 17b-CD4i epitope, in contrast to 88-CD4li, may be present as a consequence of dramatic conformational changes after CD4 binding to gp120, b) in the absence of CD4 binding, the gp120 V3 region may mask the 17b-CD4i epitope better that the S8-CD4il epitope, or c) a combination of these processes.

The inventor's model suggests that S8 Fab may bind to two gp120 regions, Ile420-Gln422 and Pro437-Pro438, located in different chains of the gp120 structure that form part of the bridging sheet minidomain. The importance of HCDR3 in the Ab-Ag interaction has been described (Morea, *et al.,* 1997). Results from mutagenesis of Arg95 in the HCDR3 confirm our previous model showing that this HCDR3 residue is fundamental in Fab S8 binding of gp120 (Torán, *et al.,* 1999). In light of these results, they searched the putative gp120 epitope for amino acid residues able to interact with the Fab S8 HCDR3 loop, and found Glu381 as the only candidate to establish an electrostatic interaction with Arg95. Glu381 interaction with Lys207 forms a salt bridge between the inner and outer gp120 domains (Rizzuto and Sodroski, 2000); changes in Glu381 or Lys207 abrogate CCR5 binding, indicating the importance of this interdomain relationship for interaction with the coreceptor (Rizzuto, *et al.,* 1998). A hypothetical interaction between Arg95 in the Fab S8 HCDR3 loop and Glu381 in gp120 may thus result in relevant changes in the gp120 inner-outer interdomain relationships.

Finally, the inventors analyzed Fab S8 epitope variability from a large non-redundant alignment of gp120 amino acids. Their results indicate low variability for amino acids Glu381, Ile420, Gln422, Pro437, and Pro438, indicating a high degree of conservation for the S8 epitope among HIV-1 viruses. Although the HIV-1 strain specificity for chemokine co-receptors is complex, CCR5 and CXCR4 specificity is proposed to reside in the V3 variable loop of HIV-1 gp120, as a single amino acid replacement in this loop alters viral tropism (Hu, *et al.,* 2000). Our results suggest that the epitope recognized by Fab S8 is not V3 region-dependent. This Fab neutralized both X4 and R5 HIV-1 strains, supporting the implication of a common gp120 region in chemokine co-receptor interaction. Recent structural data show that the neutralizing face on gp120 occupies a reduced area on the molecule (Wyatt, *et al.,* 1998). Most of the potent cross-clade neutralizing mAb described (b12, 2G12 and 2F5) (Burton, *et al.,* 1994; Muster, *et al.,* 1993; Trkola, *et al.,* 1996) are directed against conformational epitopes, although these specificities are rarely induced. The Fab S8 epitope was found to be accessible on the molecule surface and conserved in most HIV-1 viruses. Interestingly, Fab S8 heavy chain is encoded by the VH3 family, a VH Ig family found to decrease in most HIV-1-infected individuals who progress to AIDS (Juompan, *et al.,* 1998). These antibody specificities may be an important factor contributing to the healthy state, and further experiments are needed to analyze the extent of S8 epitope specificity in HIV-1-infected persons. In addition, human Fab S8 could be included in antibody strategies to combat HIV-1 infection. The peptide described here, derived from the mapping of this human Fab, may contribute to understanding gp120-co-receptor interactions and development of new strategies to combat AIDS.

### Abbreviations

- CD4i: CD4-induced
- CD4il: CD4-induced-like
- CDR: complementarity-determining region
- CPE: cytopathic effect
- FR: framework region
- HC: heavy chain
- HCDR3: heavy chain complementarity-determining region 3
- IRA: infectivity reduction assay
- LC: light chain
- LTNP: long-term nonprogressor individual
- NPA: neutralization plaque assay
- NSI: non-syncytium-inducing
- PBMC: peripheral blood mononuclear cells
- PHA: phytohemagglutinin
- SI: syncytium-inducing
- TCA: T cell-adapted
- TCID: 50% tissue culture infective dose
- TCLA: T cell-adapted laboratory strains

### References

Barbas, C.R., T.A. Collet, W. Amberg, P. Roben, J.M. Binley, D. Hoekstra, D. Cababa, T.M. Jones, R.A. Williamson, G.R. Pilkington, N.L. Haigwood, E. Cabezas, A.C. Satterthwait, I. Sanz and DR. Burton. 1993. Molecular profile of an antibody response to HIV-1 as probed by combinatorial libraries. *J. Mol. Biol.,* **230**, 812-823.

Bateman, A., E. Birney, R. Durbin, S.R. Eddy, K.L. Howe and E.L. Sonnhammer. 2000. The Pfam Protein Families Database. *Nucleic Acids Res.,* **28**, 263-266.

Beddows, S., S. Lister, R. Cheingsong, C. Bruck and J. Weber. 1999. Comparison of the antibody repertoire generated in healthy volunteers following immunization with a monomeric recombinant gp120 construct derived from a CCR5/CXCR4-using human immunodeficiency virus type 1 isolate with sera from naturally infected individuals. J. *Virol.,* **73**, 1740-1745.

Berger, E.A., P.M. Murphy and J.M. Farber. 1999. Chemokine receptors as HIV-1 coreceptors: roles in viral entry, tropism, and disease. *Annu. Rev. Immunol.,* **17**, 657-700.

Boots, L.J., P.M. McKenna, B.A. Arnold, P.M. Keller, M.K. Gorny, P.S. Zolla, J.E. Robinson and A.J. Conley. 1997. Anti-human immunodeficiency virus type 1 human monoclonal antibodies that bind discontinuous epitopes in the viral glycoproteins can identify mimotopes from recombinant phage peptide display libraries. *AIDS Res. Hum. Retrovirus,* **13**, 1549-1559.

Burton, D.R., J. Pyati, R. Koduri, S.J. Sharp, G.B. Thornton, P.W. Parren, L.S. Sawyer, R.M. Hendry, N. Dunlop, P.L. Nara, M. Lamacchia, E. Garratty, E.R. Stiehm, Y.J. Bryson, Y. Cao, J.P. Moore, D.D. Ho and C.F. Barbas III. 1994. Efficient neutralization of primary isolates of HIV-1 by a recombinant human monoclonal antibody. *Science,* **266**, 1024-1027.

Casado, C., I. Urtasun, W.M. Martin, S. Garcia, C. Rodriguez, J. del Romero and G.C. Lopez. (2000a). Genetic analysis of HIV-1 samples from Spain. *J. Acquir. Immune* Defic. *Syndr.,* **23**, 68-74.

Casado, C., I. Urtasun, S. Saragosti, M.L. Chaix, A. De Rosi, A.M. Cattelan, U. Dietrich and G.C. Lopez. (2000b). Different distribution of HIV type 1 genetic variants in European patients with distinct risk practices. *AIDS Res. Hum. Retrovirus ,* **16**, 299-304.

Collet, T.A., P. Roben, R. O'Kennedy, C.R. Barbas, D.R. Burton and R.A. Lerner. 1992. A binary plasmid system for shuffling combinatorial antibody libraries. *Proc. Natl. Acad. Sci. USA,* **89**, 10026-10030.

Connor, R.I., K.E. Sheridan, D. Ceradini, S. Choe and N.R. Landau. 1997. Change in coreceptor use coreceptor use correlates with disease progression in HIV-1-infected individuals. *J Exp. Med.,* **185**, 621-628.

De Jong, J., A. De Ronde, W. Keulen, M. Tersmette and J. Goudsmit. 1992. Minimal requirements for the human immunodeficiency virus type 1 V3 domain to support the syncytium-inducing phenotype: analysis by single amino acid substitution. *J. Virol.,* **66**, 6777-6780.

Ditzel, H.J., P.W. Parren, J.M. Binley, J. Sodroski, J.P. Moore, C.R. Barbas and D.R. Burton. 1997. Mapping the protein surface of human immunodeficiency virus type 1 gp120 using human monoclonal antibodies from phage display libraries. *J. Mol. Biol.,* **267**, 684-695.

Felsenstein, J. 1993. PHYLIP (phylogeny interference package). Seattle, Dept of Genetics, University of Washington.

Ferrer, M. and S.C. Harrison. 1999. Peptide ligands to human immunodeficiency virus type 1 gp120 identified from phage display libraries. *J. Virol.,* **73**, 5795-5802.

Ferrer, M., B.J. Sullivan, K.L. Godbout, E. Burke, H.S. Stump, J. Godoy, A. Golden, A.T. Profy and M. Van Schravendijk. 1999. Structural and functional characterization of an epitope in the conserved C-terminal region of HIV-1 gp120. J. *Pept. Res.,* **54**, 32-42.

Harada, S., Y. Koyanagi and N. Yamamoto. 1985. Infection of HTLV-III/LAV in HTLV-I-carrying cells MT-2 and MT-4 and application in a plaque assay. *Science,* **229**, 563-566.

Hendsch, Z.S. and B. Tidor. 1994. Do salt bridges stabilize proteins? A continuum electrostatic analysis. *Protein Sci.,* **3**, 211-226.

Juompan, L., P. Lambin and M. Zouali. 1998. Selective deficit in antibodies specific for the superantigen binding site of gp120 in HIV infection. *FASEB J.,* **14**, 1473-1480.

Koot, M., A.H. Vos, R.P. Keet, R.E. de Goede, M.W. Dercksen, F.G. Terpstra, R.A. Coutinho, Miedema and M. Tersmette. 1992. HIV-1 biological phenotype in long-term infected individuals evaluated with an MT-2 cocultivation assay. AIDS, **6**, 49-54.

Kumar, S., K.Tamura and M. Nei. 1993. MEGA: molecular evolutionary genetics analysis. (University Park, PA. Pennsylvania State University).

Kwong, P.D., R. Wyatt, S. Majeed, J. Robinson, R.W., Sweet, J. Sodroski and W.A. Hendrickson. 2001. Structures of HIV-1 gp120 envelope glycoproteins from laboratory-adapted and primary isolates. *Structure Fold. Des.,* **8**, 1329-1339.

Kwong, P.D., R. Wyatt, J., Robinson, R.W. Sweet, J. Sodroski and W.A. Hendrickson. 1998. Structure of an HIV gp120 envelope glycoprotein in complex with the CD4 receptor and a neutralizing human antibody. *Nature,* **393**, 648-659.

Kwong, P.D., R. Wyatt, Q.J. Sattentau, J. Sodroski and W.A. Hendrickson. 2000. Oligomeric modeling and electrostatic analysis of the gp120 envelope glycoprotein of human immunodeficiency virus. *J. Virol.,* **74**, 1961-1972.

Loomis, L.D., C.D. Deal, K.S. Kersey, D.S. Burke, R.R. Redfield and D.L. Birx. 1995. Humoral responses to linear epitopes on the HIV-1 envelope in seropositive volunteers after vaccine therapy with rgp160. J. *Acquir. Immune Defic. Syndr. Hum. Retrovirol.,* **10**, 13-26.

Mascola, J.R., S.W. Snyder, O.S. Weislow, S.M. Belay, R.B. Belshe, D.H. Schwartz, M.L. Clements, R. Dolin, B.S. Graham, G.J. Gorse, M.C. Keefer, M.J. McElrath, M.C. Walker, K.F. Wagner, J.G. McNeil, M.E. McCutchan and D.S. Burke. 1996. Immunization with envelope subunit vaccine products elicits neutralizing antibodies against laboratory-adapted but not primary isolates of human immunodeficiency virus type 1. National Institute of Allergy and Infectious Diseases AIDS Vaccine Evaluation Group. *J. Infect. Dis.,* **173**, 340-348.

McCutchan, *et al., AIDS Research and Human Retroviruses,* 8 (1992), 1887-1895.

McDermott, D.H., P.A. Zimmerman, F. Guignard, C.A. Kleeberger, S.F. Leitman and P.M. Murphy. 1998. CCR5 promoter polymorphism and HIV-1 disease progression. Multicenter AIDS Cohort Study (MACS). *Lancet,* **352**, 866-870.

McLachlan, A.D. 1971. Tests for comparing related amino-acid sequences. Cytochrome c and cytochrome c 551. *J. Mol. Biol.,* **61**, 409-424.

Morea, V., A. Tramontano, M. Rustici, C. Chothia and A.M. Lesk. 1997. Antibody structure, prediction and redesign. *Biophys. Chem.,* **68**, 9-16.

Mosier, D.E. 1996. Human immunodeficiency virus infection of human cells transplanted to severe combined immunodeficient mice. *Adv. Immunol.,* **63**, 79-125.

Muster, T., F. Steindl, M. Purtscher, A. Trkola, A. Klima, G. Himmler, F. Ruker and H. Katinger. 1993. A conserved neutralizing epitope on gp41 of human immunodeficiency virus type 1. *J. Virol.,* **67**, 6642-6647.

Myers, G., B. Korber, J.A. Berzofksy, R. Smith and G. Pavlakis. 1992. Human Retroviruses and AIDS 1992: A Compilation and Analysis of Nucleic Acid and Amino Acid Sequences.Theoretical Biology and Biophysics Group, Los Alamos National Laboratory. Theoretical Biology and Biophysics Group, N. Theoretical Biology and Biophysics Group. Los Alamos, ed.

Nicholls, A., K.A. Sharp and B. Honig. 1991. Protein folding and association: insights from the interfacial and thermodynamic properties of hydrocarbons. *Proteins ,* **11**, 281-296.

O'Brien, S.J. and J.P. Moore. 2000. The effect of genetic variation in chemokines and their receptors on HIV transmission and progression to AIDS. *Immunol. Rev.,* **177**, 99-111.

Page, R.D. 1996. TreeView: an application to display phylogenetic trees on personal computers. *Comput. Appl. Biosci.,* **12**, 357-358.

Persson, M.A., R.H. Caothien and D.R. Burton. 1991. Generation of diverse high-affinity human monoclonal antibodies by repertoire cloning. *Proc. Natl. Acad. Sci. USA,* **88**, 2432-2436.

Ratner, L. *et al., Nature,* **313** (1985), 277-284.

Rizzuto, C. and J. Sodroski. 2000. Fine definition of a conserved CCR5-binding region on the human immunodeficiency virus type 1 glycoprotein 120. *AIDS Res. Hum. Retrovirus,* **16**, 741-749.

Rizzuto, C.D., R. Wyatt, R.N. Hernandez, Y. Sun, P.D. Kwong, W.A. Hendrickson and J. Sodroski. 1998. A conserved HIV gp120 glycoprotein structure involved in chemokine receptor binding. *Science ,* **280**, 1949-1953.

Salzwedel, K., E.D. Smith, B. Dey and E.A. Berger. 2000. Sequential CD4-coreceptor interactions in human immunodeficiency virus type 1 Env function: soluble CD4 activates Env for coreceptor-dependent fusion and reveals blocking activities of antibodies against cryptic conserved epitopes on gp120. *J. Virol.,* **74**, 326-33.

Sanchez, P.S., J.M. Rojas, M.A. Martinez, E.M. Fenyo, R. Najera, E. Domingo and G.C. Lopez. 1993. Dilute passage promotes expression of genetic and phenotypic variants of human immunodeficiency virus type 1 in cell culture. *J. Virol.,* **67**, 2938-29343.

Schellekens, G.A., E. Lasonder, M. Feijlbrief, D.G. Koedijk, J.W. Drijfhout, A.J. Scheffer, W.S. Welling and G.W. Welling. 1994. Identification of the core residues of the epitope of a monoclonal antibody raised against glycoprotein D of herpes simplex virus type 1 by screening of a random peptide library. *Eur. J. Immunol.,* **24**, 3188-3193.

Scott, J.K. and G.P. Smith. 1990. Searching for peptide ligands with an epitope library. *Science ,* **249**, 386-390.

Shankarappa, R., J.B. Margolick, S.J. Gange, A.G. Rodrigo, D. Upchurch, H. Farzadegan, P. Gupta, C.R. Rinaldo, G.H. Learn, X. He, X.L. Huang and J.I. Mullins. 1999. Consistent viral e volutionary c hanges associated with the progression o f human immunodeficiency virus type 1 infection. *J. Virol.,* **73**, 10489-10502.

Sindelar, C.V., Z.S. Hendsch and B. Tidor. 1998. Effects of salt bridges on protein structure and design. *Protein Sci.,* **7**, 1898-1914.

Smith, M.W., M. Dean, M. Carrington, C. Winkler, G.A. Huttley, D.A. Lomb, J.J. Goedert, T.R. O'Brien, L.P. Jacobson, R. Kaslow, S. Buchbinder, E. Vittinghoff, D. Vlahov, K. Hoots, M.W. Hilgartner and S.J. O'Brien. 1997. Contrasting genetic influence of CCR2 and CCR5 variants on HIV-1 infection and disease progression. Hemophilia Growth and Development Study (HGDS), Multicenter AIDS Cohort Study (MACS), Multicenter Hemophilia Cohort Study (MHCS), San Francisco City Cohort (SFCC), ALIVE Study. *Science,* **277**, 959-965.

Sullivan, N., Y. Sun, Q. Sattentau, M. Thali, D. Wu, G. Denisova, J. Gershoni, J. Robinson, J. Moore and J. Sodroski. 1998. CD4-Induced conformational changes in the human immunodeficiency virus type 1 gp120 glycoprotein: consequences for virus entry and neutralization. *J Virol.,* **72**,4694-4703.

Thali, M., J.P. Moore, C. Furman, M. Charles, D.D. Ho, J. Robinson and J. Sodroski. 1993. Characterization of conserved human immunodeficiency virus type 1 gp120 neutralization epitopes exposed upon gp120-CD4 binding. *J. Virol.,* **67**, 3978-3988.

Thompson, J.D., D.G. Higgins and T.J. Gibson. 1994. CLUSTAL W: improving the sensitivity of progressive multiple sequence alignment through sequence weighting, position-specific gap penalties and weight matrix choice. *Nucleic Acids Res.,* **22**, 4673-4680.

Torán, J.L., L. Kremer, P.L. Sanchez, I. Moreno de Alboran, G. del Real, M. Llorente, A. Valencia, M. Alvarez de Mon and C. Martinez-A. 1999. Molecular analysis of HIV-1 gp120 antibody response using isotype IgM and IgG phage display libraries from a long-term non-progressor HIV-1-infected individual. *Eur. J. Immunol.,* **29**, 2666-2675.

Torán, J. L., P.L, Sanchez, L. Kremer, G. del Real, A. Valencia and C. Martinez-A. 2001. Improvement in affinity and HIV-1 neutralization by somatic mutation in the heavy chain first complementarity-determining region of antibodies triggered by HIV-1 infection. *Eur. J.* Immunol., **31**, 128-137.

Trkola, A., M. Purtscher, T. Muster, C. Ballaun, A. Buchacher, N. Sullivan, K. Srinivasan, J. Sodroski, J.P. Moore and H. Katinger. 1996. Human monoclonal antibody 2G12 defines a distinctive neutralization epitope on the gp120 glycoprotein of human immunodeficiency virus type 1. *J.* Virol., **70**, 1100-1108.

Vriend, G. (1990). WHAT IF: a molecular modeling and drug design program. J. *Mol. Graph.,* **8**, 52-56.

Wyatt, R., P.D. Kwong, E. Desjardins, R.W. Sweet, J. Robinson, W.A. Hendrickson and J.G. Sodroski. 1998. The antigenic structure of the HIV gp120 envelope glycoprotein. *Nature,* **393**, 705-711.

Yip, Y.L. and R.L. Ward. 1999. Epitope discovery using monoclonal antibodies and phage peptide libraries. *Comb. Chem. High Throughput Screen.,* **2**, 125-138.

## Claims

1. An antibody or a fragment thereof comprising a light chain and/or a heavy chain, the light chain or heavy chain comprising the amino acid sequence shown in SEQ ID 2, SEQ ID 4, SEQ ID 6 or SEQ ID 8, which is capable of binding gp120 protein from HIV.

2. A fragment of an antibody as defined in claim 1, the fragment being capable of binding gp120 protein of HIV with the proviso that when part of SEQ ID 4, SEQ ID 6, or SEQ ID 8 are present, at least one amino acid from amino acid number 119 of each sequence is present in the antibody fragment.

3. An antibody or a fragment thereof according to claim 1 or claim 2, comprising Arg₉₅ of an HCDR3 domain.

4. An antibody or a fragment thereof comprising a light chain and a heavy chain, the light chain comprising the amino acid sequence shown in SEQ ID 2 and the heavy chain comprising an amino acid sequence selected from SEQ ID 4, SEQ ID 6 and SEQ ID 8, the antibody or fragment being capable of binding gp120 protein from HIV.

5. An antibody fragment according to any one of claims 1-4, which is an F(ab')₂ or an Fab fragment.

6. A nucleic acid molecule selected from:
(a) a nucleic acid molecule which encodes for an antibody or a fragment of an antibody according to any preceding claim;
(b) a nucleic acid molecule comprising the nucleotide sequence shown in SEQ ID 1 and optionally one of SEQ IDS, SEQ IDS or SEQ ID 7;
(c) a nucleic acid molecule, the complementary strand of which hybridises to a nucleic acid molecule as defined in (a) or (b) and which encodes an antibody or a fragment of an antibody light chains and which is capable of binding gp120 protein from HIV; and
(d) nucleic acid molecules which differ from the sequence of (c) due to the degeneracy of the genetic code.

7. A vector comprising a nucleic acid molecule according to claim 6.

8. A host cell comprising a vector according to claim 7.

9. Use of an antibody or a fragment thereof according to any one of claims 1 to 5, to identify a chemical compound capable of competing for the binding of the antibody or fragment thereof to HIV gp120 protein or a fragment thereof.

10. A chemical compound identifiable by a method according to claim 10.

11. A compound according to claim 10 which is a peptide.

12. A peptide according to claim 11 which is a conformational epitope to one or both of regions Ile₄₂₀ - Gln₄₂₂ and/or Pro₄₃₇ - Pro₄₃₈ of the gp120 protein of HIV-1.

13. A peptide according to claim 11 or claim 12 comprising an amino acid sequence selected from: 
SEQ ID 9 | GIQLANPPRLYG
SEQ ID 10 | FLQPPDFSHLPP
SEQ ID 11 | SAMEAPP
SEQ ID 12 | LLADTTHHRPWT
SEQ ID 13 | QEVGKAMYAPPI
 or a sequence shown in any one of SEQ ID Nos. 14 to 43.

14. A peptide according to claim 13 comprising both SEQ ID 9 and SEQ ID 10.

15. A vaccine comprising a compound according to any one of claims 10 to 14.

16. An antibody or fragment thereof according to any one of claims 1 to 5 or a compound according to any one of claims 10 to 14 for use to treat HIV infections.

17. A kit for studying HIV infection *in vivo* or *in vitro* comprising an antibody or a fragment thereof according to any one of claims 1 to 5, or a compound according to any one of claims 10 to 14.

18. An isolated peptide comprising an amino acid sequence which encodes for one or both of regions Ile₄₂₀ - Gln₄₂₂ and/or Pro₄₃₇ - Pro₄₃₈ of the gp120 protein of HIV.

19. A method of inhibiting the binding of HIV to a viral co-receptor comprising the use of an antibody or a fragment according to any one of claims 1 to 5, or a chemical compound according to any one of claims 10 to 14.

20. Use of an antibody or fragment thereof according to any one of claims 1 to 5 or a compound according to any one of claims 10 to 14 to evaluate AIDS progression and/or the state of infection as a prognosis marker.
